(19) Europäisches Patentamt
European Patent Office
Office européen des brevets

(11) Veröffentlichungsnummer: **0 410 238 A2**

(12) **EUROPÄISCHE PATENTANMELDUNG**

(21) Anmeldenummer: **90113508.7**

(51) Int. Cl.5: **C07D 237/22**, A01N 43/58

(22) Anmeldetag: **14.07.90**

(30) Priorität: **28.07.89 DE 3925066**
**27.03.90 DE 4009761**

(43) Veröffentlichungstag der Anmeldung:
**30.01.91 Patentblatt 91/05**

(84) Benannte Vertragsstaaten:
**BE CH DE FR GB IT LI NL**

(71) Anmelder: **BAYER AG**

**D-5090 Leverkusen 1 Bayerwerk(DE)**

(72) Erfinder: **Weissmüller, Joachim, Dr.**
**Carl-Langhans-Strasse 53**
**D-4019 Monheim(DE)**
Erfinder: **Babczinski, Peter, Dr.**
**In der Lohrenbeck 11**
**D-5600 Wuppertal(DE)**
Erfinder: **Lürssen, Klaus, Dr.**
**August-Kierspel-Strasse 145**
**D-5060 Bergisch-Gladbach 2(DE)**
Erfinder: **Santel, Hans-Joachim, Dr.**
**Gruenstrasse 9a**
**D-5090 Leverkusen(DE)**
Erfinder: **Schmidt, Robert R., Dr.**
**Im Waldwinkel 110**
**D-5060 Bergisch-Gladbach 2(DE)**
Erfinder: **Krauskopf,Birgit,Dr.**
**Kicke 19**
**D-5060 Bergisch-Gladbach 1(DE)**

(54) **2H-Pyridazinon-Derivate.**

(57) Die Erfindung betrifft neue 2H-Pyridazinon-Derivate der allgemeinen Formel (I),

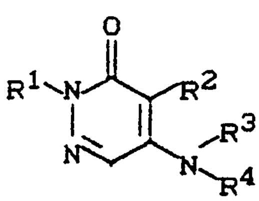

(I)

in welcher
$R^1$ für Alkyl, Alkoxyalkyl, Alkylthioalkyl, Aminoalkyl, Alkylaminoalkyl, Dialkylaminoalkyl, Hydroxyalkyl, Halogenalkyl, Cyanalkyl, Alkenyl, Halogenalkenyl, Alkoxycarbonylalkyl oder für jeweils gegebenenfalls substituiertes Cycloalkyl, Cycloalkylalkyl, Phenyl oder Phenylalkyl steht,
$R^2$ für Alkyl oder für jeweils gegebenenfalls substituiertes Phenyl, Phenoxy oder Phenylalkyl steht,

EP 0 410 238 A2

## 2H-PYRIDAZINON-DERIVATE

Die Erfindung betrifft neue 2H-Pyridazinon-Derivate, Verfahren zu ihrer Herstellung und ihre Verwendung als Herbizide und/oder Pflanzenwachstumsregulatoren.

Es ist bekannt, daß bestimmte Stickstoffheterocyclen wie beispielsweise das 2-(3-Trifluormethyl-phenyl)-4-chlor-5-methylamino-2H-pyridazin-3-on (vgl. DE-OS 1695840) sowie bestimmte Furanone, wie beispielsweise das 5-(Methylamino)-2-phenyl-4-[3-(trifluormethyl)-phenyl]-2H-furan-3-on (vgl. DE-OS 3422346) herbizide Eigenschaften besitzen.

Die herbizide Wirksamkeit dieser vorbekannten Verbindungen gegenüber Problemunkräutern ist jedoch ebenso wie ihre Verträglichkeit gegenüber wichtigen Kulturpflanzen nicht in allen Anwendungsgebieten völlig zufriedenstellend.

Es wurden neue 2H-Pyridazinon-Derivate der allgemeinen Formel (I),

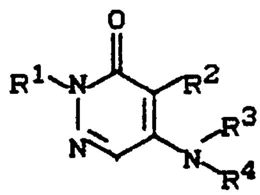

(I)

in welcher
$R^1$ für Alkyl, Alkoxyalkyl, Alkylthioalkyl, Aminoalkyl, Alkylaminoalkyl, Dialkylaminoalkyl, Hydroxyalkyl, Halogenalkyl, Cyanalkyl, Alkenyl, Halogenalkenyl, Alkoxycarbonylalkyl oder für jeweils gegebenenfalls substituiertes Cycloalkyl, Cycloalkylalkyl, Phenyl oder Phenylalkyl steht,
$R^2$ für Alkyl oder für jeweils gegebenenfalls substituiertes Phenyl, Phenoxy oder Phenylalkyl steht,
$R^3$ für Wasserstoff oder für jeweils gegebenenfalls substituiertes Alkyl, Cycloalkyl oder Alkylcarbonyl steht und
$R^4$ für Wasserstoff, Hydroxy, Amino, Aminocarbonyl (Carbamoyl), für Alkoxy, Alkylamino oder Dialkylamino, für jeweils gegebenenfalls substituiertes Alkyl, Cycloalkyl, Alkylaminocarbonyl, Dialkylaminocarbonyl, Alkylcarbonyloxy, Alkyl carbonylamino, Alkylcarbonylalkylamino, Bis-(alkylcarbonyl)-amino, Alkoxycarbonyl, Alkoxycarbonylcarbonyl, Formyl oder Alkylcarbonyl steht,
gefunden.

Weiterhin wurde gefunden, daß man die neuen 2H-Pyridazinon-Derivate der allgemeinen Formel (I) erhält, wenn man

(a) 2H-5-Halogen-pyridazinon-Derivate der allgemeinen Formel (II),

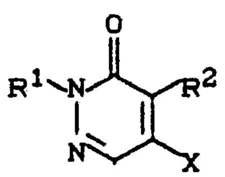

(II)

in welcher
$R^1$ und $R^2$ die oben angegebene Bedeutung haben und
X für Halogen steht,
mit Aminen der allgemeinen Formel (III),

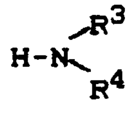

(III)

in welcher
$R^3$ und $R^4$ die oben angegebene Bedeutung haben,

gegebenenfalls in Gegenwart eines Verdünnungsmittels und gegebenenfalls in Gegenwart eines basischen Reaktionshilfsmittels (Säurebindemittels) umsetzt, oder

(b) die nach Verfahren (a) erhaltenen erfindungsgemäßen Verbindungen der allgemeinen Formel (Ia),

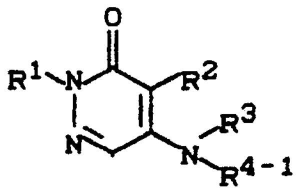

(Ia)

in welcher

$R^1$, $R^2$ und $R^3$ die oben angegebene Bedeutung haben und

$R^{4-1}$ für Wasserstoff, Hydroxy, Amino oder Alkylamino steht,

mit Acylierungsmitteln der allgemeinen Formel (IV),

$R^{4-2}$-Z (IV)

in welcher

$R^{4-2}$ für jeweils gegebenenfalls substituiertes (Di)Alkylaminocarbonyl, Alkoxycarbonyl, Alko xycarbonylcarbonyl oder Alkylcarbonyl steht und

Z für eine elektronenanziehende Abgangsgruppe steht,

gegebenenfalls in Gegenwart eines Verdünnungsmittels und gegebenenfalls in Gegenwart eines basischen Reaktionshilfsmittels (Säurebindemittels) umsetzt.

Schließlich wurde gefunden, daß die neuen 2H-Pyridazinon-Derivate der allgemeinen Formel (I) herbizide und wachstumsregulierende Eigenschaften besitzen.

Überraschenderweise zeigen die erfindungsgemäßen 2H-Pyridazinon-Derivate der allgemeinen Formel (I) eine erheblich höhere herbizide Potenz gegenüber Problemunkräutern als das aus dem Stand der Technik bekannte, strukturverwandte 5-Methylamino-2-phenyl-4-[3-(trifluormethyl)-phenyl]-2H-furan-3-on, welches auch wirkungsmäßig eine naheliegende Verbindung darstellt. Neuartig und überraschend ist die wachstumsregulierende Wirkung einiger erfindungsgemäßer 2H-Pyridazinon-Derivate der Formel (I).

Die erfindungsgemäßen 2H-Pyridazinon-Derivate sind durch die Formel (I) allgemein definiert. Bevorzugt sind Verbindungen der Formel (I), bei welchen

$R^1$ für geradkettiges oder verzweigtes Alkyl mit 1 bis 6 Kohlenstoffatomen, geradkettiges oder verzweigtes Alkoxyalkyl oder Alkylthioalkyl mit jeweils 1 bis 4 Kohlenstoffatomen im Alkyl- und Alkoxy- bzw. Alkylthioteil, geradkettiges oder verzweigtes Aminoalkyl, Alkylaminoalkyl, Dialkylaminoalkyl, Hydroxyalkyl, Halogenalkyl oder Cyanalkyl mit jeweils 1 bis 4 Kohlenstoffatomen in den jeweiligen Alkylteilen und 1-4 Halogenatomen im Halogenalkylteil, geradkettiges oder verzweigtes Alkenyl oder Halogenalkenyl mit jeweils 2-6 Kohlenstoffatomen im Alkenylteil und 1-4 Halogenatomen im Halogenalkenylteil, geradkettiges oder verzweigtes Alkoxycarbonylalkyl mit jeweils 1-4 Kohlenstoffatomen im Alkoxy- und Alkylteil, gegebenenfalls einfach bis vierfach gleich oder verschieden im Cycloalkylteil substituiertes Cycloalkyl oder Cycloalkylalkyl mit jeweils 3-6 Kohlenstoffatomen im Cycloalkylteil und gegebenenfalls 1-3 Kohlenstoffatomen im geradkettigen oder verzweigten Alkylteil steht (wobei als Substituenten jeweils infrage kommen: Halogen, jeweils geradkettiges oder verzweigtes Alkyl, Alkoxy, Halogenalkyl oder Halogenalkoxy mit jeweils 1 bis 4 Kohlenstoffatomen und gegebenenfalls 1 bis 9 gleichen oder verschiedenen Halogenatomen); oder für gegebenenfalls einfach bis dreifach gleich oder verschieden im Phenylteil substituiertes Phenyl oder Phenylalkyl mit gegebenenfalls 1-3 Kohlenstoffatomen im geradkettigen oder verzweigten Alkylteil steht (wobei als Substituenten jeweils in frage kommen: Halogen, Cyano, Nitro, jeweils geradkettiges oder verzweigtes Alkyl oder Alkoxy mit jeweils 1-6 Kohlenstoffatomen, Halogenalkyl oder Halogenalkoxy mit jeweils 1 oder 2 Kohlenstoffatomen und 1-5 gleichen oder verschiedenen Halogenatomen);

$R^2$ für geradkettiges oder verzweigtes Alkyl mit 1-6 Kohlenstoffatomen oder für gegebenenfalls einfach bis dreifach gleich oder verschieden im Phenylteil substituiertes Phenyl, Phenoxy oder Phenylalkyl mit gegebenenfalls 1-3 Kohlenstoffatomen im geradkettigen oder verzweigten Alkylteil steht (wobei als Substituenten jeweils infrage kommen: Halogen, Cyano, Nitro, jeweils geradkettiges oder verzweigtes Alkyl oder Alkoxy mit jeweils 1-6 Kohlenstoffatomen, jeweils geradkettiges oder verzweigtes Halogenalkyl oder Halogenalkoxy mit jeweils 1-2 Kohlenstoffatomen und 1-5 gleichen oder verschiedenen Halogenatomen);

$R^3$ für Wasserstoff, für jeweils geradkettiges oder verzweigtes Alkyl mit 1-4 Kohlenstoffatomen (welches gegebenenfalls durch 1-9 gleiche oder verschiedene Halogenatome und/oder durch Alkoxy mit 1-4 Kohlenstoffatomen substituiert ist), oder für gegebenenfalls einfach bis vierfach gleich oder verschieden substituiertes Cycloalkyl mit 3-6 Kohlenstoffatomen steht (wobei als Substituenten jeweils infrage kommen: Halogen, sowie jeweils ge radkettiges oder verzweigtes Alkyl, Alkoxy, Halogenalkyl oder Halogenalkoxy mit jeweils 1-4 Kohlenstoffatomen und gegebenenfalls 1-9 gleichen oder verschiedenen Halogenatomen), oder für geradkettiges oder verzweigtes und gegebenenfalls substituiertes Alkylcarbonyl mit 1-4 Kohlenstoffatomen im Alkylteil steht (wobei als Substituenten 1-3 gleiche oder verschiedene Halogenatome infrage kommen),

und

$R^4$ für Wasserstoff, Hydroxy, Amino, Aminocarbonyl, für jeweils geradkettiges oder verzweigtes Alkoxy, Alkylamino oder Dialkylamino mit 1-4 Kohlenstoffatomen in den jeweiligen Alkylteilen, für jeweils geradkettiges oder verzweigtes Alkyl mit 1-4 Kohlenstoffatomen (welches gegebenenfalls durch 1-9 gleiche oder verschiedene Halogenatome und/oder Hydroxygruppen bzw. durch Alkoxy mit 1-4 Kohlenstoffatomen substituiert ist) oder für gegebenenfalls einfach bis vierfach gleich oder verschieden substituiertes Cycloalkyl mit 3-6 Kohlenstoffatomen (wobei als Substituenten jeweils infrage kommen: Halogen, sowie jeweils geradkettiges oder verzweigtes Alkyl, Alkoxy, Halogenalkyl oder Halogenalkoxy mit jeweils 1-4 Kohlenstoffatomen und gegebenenfalls 1-9 gleichen oder verschiedenen Halogenatomen), für jeweils geradkettiges oder verzweigtes Alkylaminocarbonyl oder Dialkylaminocar bonyl mit 1-4 Kohlenstoffatomen in den jeweiligen Alkylteilen, für im Alkylcarbonylteil gegebenenfalls jeweils 1-3fach durch Halogen substituiertes Alkylcarbonyloxy, Alkylcarbonylamino, Alkylcarbonyl-alkyl-amino oder Bis-(alkylcarbonyl)-amino mit 1-4 Kohlenstoffatomen in den jeweiligen Alkylteilen, für jeweils geradkettiges oder verzweigtes und gegebenenfalls substituiertes Alkoxycarbonyl oder Alkoxycarbonylcarbonyl mit 1-4 Kohlenstoffatomen im Alkylteil oder für geradkettiges oder verzweigtes und gegebenenfalls substituiertes Alkylcarbonyl mit 1-4 Kohlenstoffatomen im Alkylteil steht (wobei als Substituenten 1-3 gleiche oder verschiedene Halogenatome infrage kommen).

Besonders bevorzugt sind Verbindungen der Formel (I), bei welchen

$R^1$ für Methyl, Ethyl, n- oder i-Propyl, n-, i-, s-oder t-Butyl, Methoxymethyl, Ethoxymethyl, Methoxyethyl oder Ethoxyethyl, Methylthiomethyl, Ethylthiomethyl, Methylthioethyl, Ethylthioethyl, Aminomethyl, Aminoethyl, Methylaminomethyl, Methylaminoethyl, Dimethylaminomethyl, Dimethylaminoethyl, Ethylaminomethyl, Ethylaminoethyl, Diethylaminomethyl, Diethylaminoethyl, Hydroxymethyl, Hydroxyethyl, 1-3 Fluor- und/oder Chloratome enthaltendes Halogenmethyl, Halogenethyl, n- oder i-Halogenpropyl, n- oder i-Halogenbutyl, Cyanmethyl, Cyanethyl, Allyl, n- oder i-Butenyl n- oder i-Pen tenyl, 2-Fluorpropen-3-yl, 2-Chlorpropen-3-yl, 1-Chlorpropen-3-yl, 1,1-Dichlorpropen-3-yl, 1-Fluorpropen-3-yl, 1,1-Difluorpropen-3-yl, 1,2-Dichlorpropen-3-yl, 1,2-Difluorpropen-3-yl, 1,1,2-Trichlorpropen-3-yl, für Methoxycarbonylmethyl, Methoxycarbonylethyl, Ethoxycarbonylmethyl oder Ethoxycarbonylethyl, oder für gegebenenfalls ein- bis vierfach gleich oder verschieden im Cycloalkylteil substituiertes Cyclopentyl, Cyclohexyl, Cyclopropylmethyl, Cyclopentylmethyl, Cyclopentylethyl, Cyclohexylmethyl oder Cyclohexylethyl steht (wobei als Substituenten jeweils infrage kommen: Fluor, Chlor, Brom, Methyl, Ethyl, Methoxy, Ethoxy, Halogenmethyl, Halogenethyl, Halogenmethoxy oder Halogenethoxy, wobei Halogen bevorzugt für Fluor und/oder Chlor steht); weiterhin für gegebenenfalls ein- oder zweifach gleich oder verschieden im Phenylteil substituiertes Phenyl, Benzyl oder Phenethyl steht (wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, Trifluormethyl, Difluormethyl sowie Trifluormethoxy);

$R^2$ für Methyl, Ethyl, n- oder i-Propyl, n-, i-, s-oder t-Butyl, oder für gegebenenfalls ein- bis zweifach gleich oder verschieden im Phenylteil substituiertes Phenyl, Phenoxy, Benzyl oder Phenethyl steht (wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, Trifluormethyl, Difluormethyl sowie Trifluormethoxy);

$R^3$ für Wasserstoff, Methyl, Ethyl, n- oder i-Propyl, Halogenmethyl oder Halogenethyl (wobei Halogen bevorzugt für Fluor und/oder Chlor steht), Methoxymethyl, Methoxyethyl, Ethoxymethyl, Ethoxyethyl, für gegebenenfalls ein- oder zweifach gleich oder verschieden substituiertes Cyclopropyl (wobei als Substituenten jeweils infrage kommen: Fluor, Chlor, Brom, Methyl, Ethyl) oder für Acetyl, Formyl, Propionyl, Trifluoracetyl, Trichloracetyl oder Chloracetyl steht, und

$R^4$ für Wasserstoff, Hydroxy, Amino, Methoxy, Ethoxy, Methylamino, Ethylamino, Dimethylamino, Diethylamino, Methyl, Ethyl, n- oder i-Propyl, Hydroxymethyl, Hydroxyethyl, Methylaminocarbonyl, Ethylaminocarbonyl, Dimethylaminocarbonyl, Methylethylaminocarbonyl oder Diethylaminocarbonyl, Acetyloxy, Trifluoracetyloxy, Acetylamino, Trifluoracetylamino, Acetylmethylamino, Trifluoracetyl-methylamino, Diacetylamino, Di-trifluoracetylamino, Methoxycarbonyl, Ethoxycarbonyl, Methoxycarbonylcarbonyl, Ethoxycarbonylcarbonyl, Formyl, Acetyl, Propionyl, Difluoracetyl, Trifluoracetyl, Chloracetyl, Dichloracetyl oder Trichloracetyl steht.

Ganz besonders bevorzugt sind Verbindungen der Formel (I), bei welchen

$R^1$ für Methyl, Ethyl, n- oder i-Propyl, n-, i-, s-oder t-Butyl, Methoxymethyl, Ethoxymethyl, Methoxyethyl,

Methylthiomethyl, Ethylthiomethyl, Methylthioethyl, Dimethylaminomethyl, Hydroxymethyl, 1,1,1-Trifluorethyl, Allyl, n- oder i-Butenyl, n-oder i-Pentenyl, 2-Fluorpropen-3-yl, 2-Chlorpropen-3-yl, 1-Chlorpropen-3-yl, 1,1-Dichlorpropen-3-yl, 1-Fluorpropen-3-yl, 1,1-Difluorpropen-3-yl, 1,2-Dichlorpropen-3-yl, 1,2-Difluorpropen-3-yl, 1,1,2-Trichlorpropen-3-yl, Cyclopropylmethyl, 1,1-Dichlorcyclopropylmethyl, 1,1-Difluorcyclopropylmethyl, 1,1-Dimethylcyclopropylmethyl, 1,1-Dimethyl-2,2-dichlorcyclopropylmethyl, für jeweils gegebenenfalls einfach oder zweifach substituiertes Cyclopentyl oder Cyclohexyl steht (wobei als Substituenten Methyl und/oder Ethyl infrage kommen), oder für gegebenenfalls einfach oder zweifach gleich oder verschieden substituiertes Phenyl steht, (wobei als Substituenten infrage kommen: Fluor, Chlor, Brom, Methyl, Ethyl, Methoxy, Ethoxy, Trifluormethyl oder Trifluormethoxy);

$R^2$ für Methyl, Ethyl n- oder i-Propyl, n-, i-, s-oder t-Butyl oder für einfach oder zweifach substituiertes Phenyl steht, wobei als Substituenten infrage kommen: Fluor, Chlor, Brom, Methyl, Ethyl, Methoxy, Ethoxy, Trifluormethyl oder Trifluormethoxy,

$R^3$ für Wasserstoff, Methyl, Ethyl, n- oder i-Propyl, Acetyl oder Trifluoracetyl steht und

$R^4$ für Hydroxy, Amino, Methylamino, Dimethylamino, Methyl, Ethyl, n- oder i-Propyl, Acetyloxy, Trifluoracetyloxy, Acetylamino, Trifluoracetylamino, Acetylmethylamino, Trifluoracetylmethylamino, Diacetylamino, Ditrifluoracetylamino, Acetyl oder Trifluoracetyl steht.

Im einzelnen seien außer den bei den Herstellungsbeispielen genannten Verbindungen der folgenden 2H-Pyridazinon-Derivate der allgemeinen Formel (I) genannt:

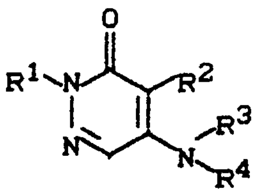

(I)

**Tabelle 1**

| $R^1$ | $R^2$ | $R^3$ | $R^4$ |
|---|---|---|---|
| 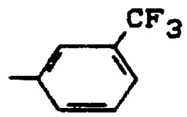 | 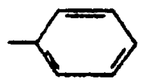 | H | $-COCH_3$ |
| 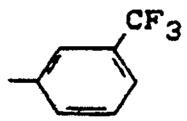 | 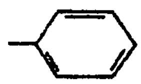 | H | $-CH_2-CH_2-OCH_3$ |
| 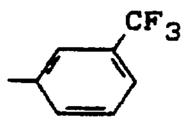 | 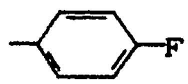 | H | $-CH_3$ |
| 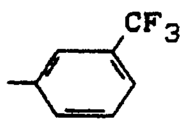 | 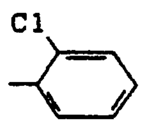 | H | $-CH_3$ |
| 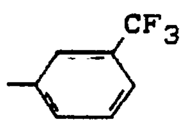 | 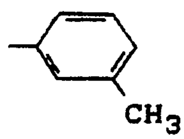 | $-CO-CH_3$ | $-CH_3$ |
| 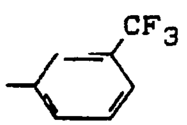 | 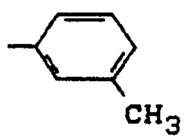 | $CH_3$ | $-CO-CF_3$ |
| 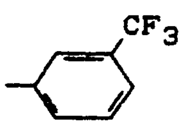 | 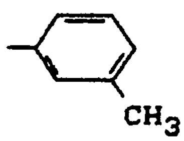 | H | $-NHCH_3$ |

Tabelle 1 - Fortsetzung

| $R^1$ | $R^2$ | $R^3$ | $R^4$ |
|---|---|---|---|
| 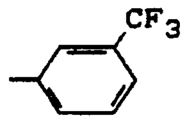 | 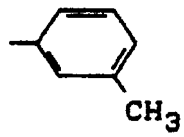 | H | 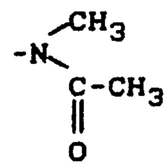 |
| 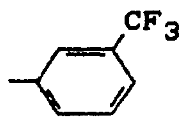 | 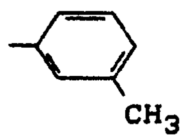 | H | -OH |
| 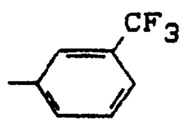 | 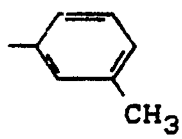 | $-CO-CH_3$ | $-O-CO-CH_3$ |
| 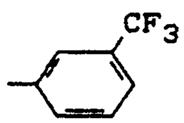 | 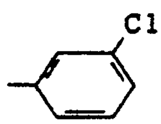 | H | $-CH_3$ |
| 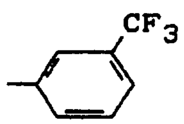 | 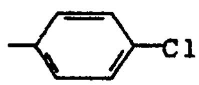 | H | $-CH_3$ |
| 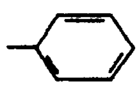 | 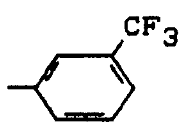 | H | $-CH_3$ |

Tabelle 1 - Fortsetzung

| $R^1$ | $R^2$ | $R^3$ | $R^4$ |
|---|---|---|---|
| 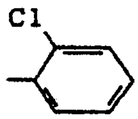 | 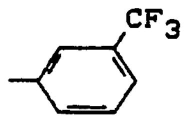 | H | $-CH_3$ |
| 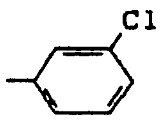 | 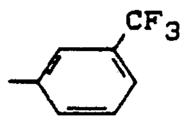 | H | $-CH_3$ |
| 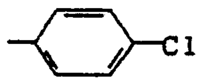 | 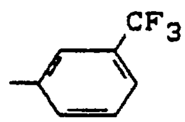 | H | $-CH_3$ |
| 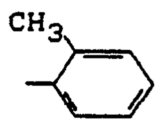 | 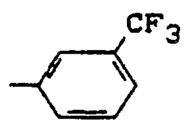 | H | $-CH_3$ |
| 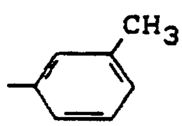 | 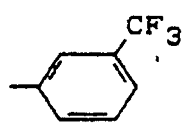 | H | $-CH_3$ |
| 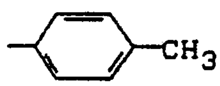 | 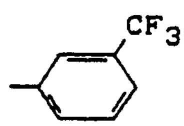 | H | $-CH_3$ |
| 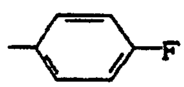 | 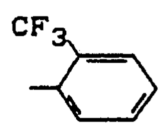 | H | $-CH_3$ |
| 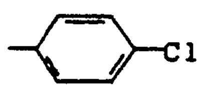 | 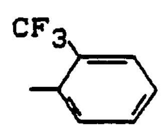 | H | $-CH_3$ |

Tabelle 1 - Fortsetzung

| $R^1$ | $R^2$ | $R^3$ | $R^4$ |
|---|---|---|---|
| 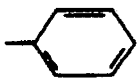 | 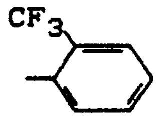 | H | $-CH_3$ |
| $-CH_3$ | 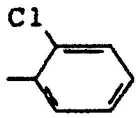 | H | $-CH_3$ |
| $-CH_3$ | 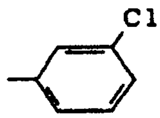 | H | $-CH_3$ |
| $-CH_3$ | 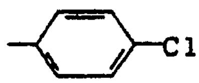 | H | $-CH_3$ |
| $-CH_3$ | 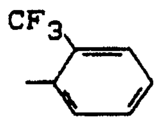 | H | $-CH_3$ |
| $-CH_3$ | 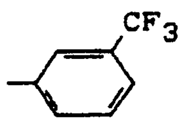 | H | $-CH_3$ |
| $-CH_3$ | 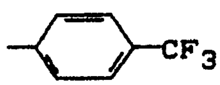 | H | $-CH_3$ |
| $-CH_2CH_2CH_3$ | 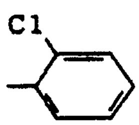 | H | $-CH_3$ |

**Tabelle 1** - Fortsetzung

| $R^1$ | $R^2$ | $R^3$ | $R^4$ |
|---|---|---|---|
| $-CH_2CH_2CH_3$ | 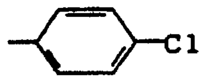 | H | $-CH_3$ |
| $-CH_2CH_2CH_3$ | 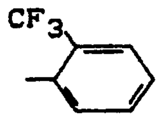 | H | $-CH_3$ |
| $-CH_2CH_2CH_3$ | 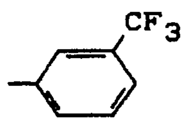 | H | $-CH_3$ |
| $-CH_2CH_2CH_3$ | 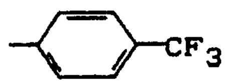 | H | $-CH_3$ |
| $-CH_2CH_2CH_3$ | 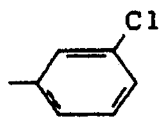 | H | $-CH_3$ |
| $-CH_2$ 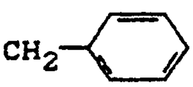 | 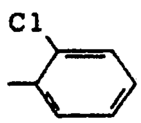 | H | $-CH_3$ |
| $-CH_2$ 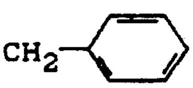 | 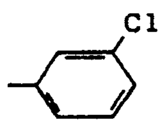 | H | $-CH_3$ |
| $-CH_2$ 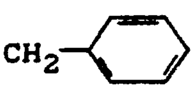 | 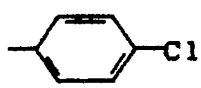 | H | $-CH_3$ |
| $-CH_2$ 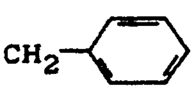 | 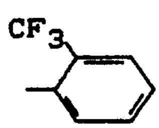 | H | $-CH_3$ |

<u>Tabelle 1</u> - Fortsetzung

| $R^1$ | $R^2$ | $R^3$ | $R^4$ |
|---|---|---|---|
| $-CH_2$ 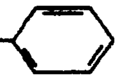 | 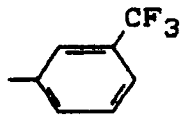 | H | $-CH_3$ |
| $-CH_2$ 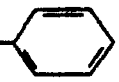 | 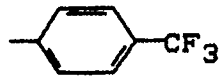 | H | $-CH_3$ |
| 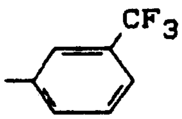 | $-CH_2CH_2CH_3$ | H | $-CH_3$ |
| 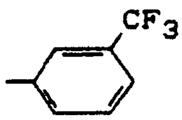 | $-CH_3$ | H | $-CH_3$ |
| 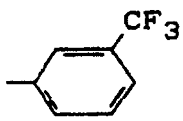 | $-CH_2$ 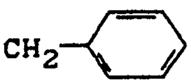 | H | $-CH_3$ |
| 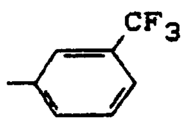 | 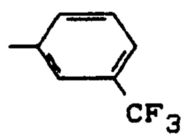 | H | $-CH_2CH_2OH$ |
| 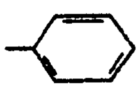 | 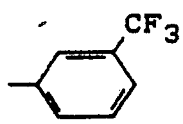 | H | $-COCH_3$ |
| 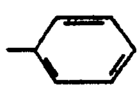 | 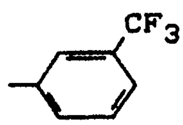 | H | $-COCF_3$ |

<u>Tabelle 1</u> - Fortsetzung

| $R^1$ | $R^2$ | $R^3$ | $R^4$ |
|---|---|---|---|
| 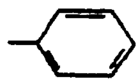 | 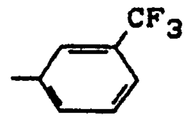 | H | $-\overset{O}{\overset{\Vert}{C}}-COOC_2H_5$ |
| $-CH_3$ | 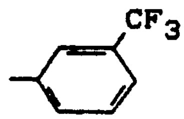 | H | $-COCH_3$ |
| $-CH_2CH_2CH_3$ | 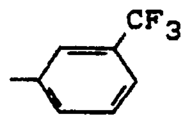 | H | $-COCH_3$ |
| $-CH_2$- 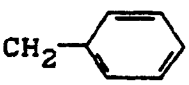 | 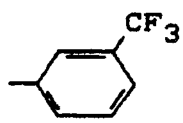 | H | $-COCH_3$ |
| 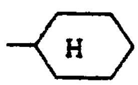 | 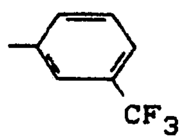 | H | $-CH_3$ |
| $-CH_2-CH{=}CH_2$ | 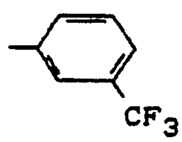 | $CH_3$ | $-COCH_3$ |
| $-CH_2-CH{=}CH_2$ | 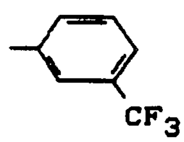 | $CH_3$ | $-COCF_3$ |
| $-CH_2-CH{=}CH_2$ | 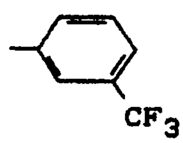 | $CH_3$ | $-OH$ |

**Tabelle 1** - Fortsetzung

| $R^1$ | $R^2$ | $R^3$ | $R^4$ |
|---|---|---|---|
| $-CH_2-CH=CH_2$ | 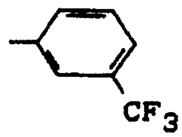 | H | $-NHCH_3$ |
| $-CH_2-CH=CH_2$ | 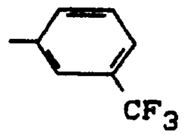 | $CH_3$ | $-NHCH_3$ |
| 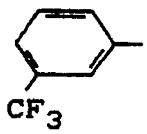 | 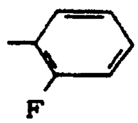 | $CH_3$ | H |
| 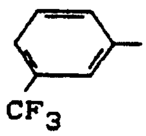 | 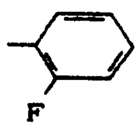 | $CH_3$ | $COCF_3$ |
| 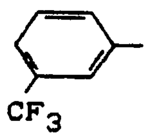 | 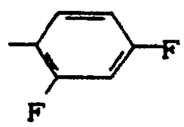 | $CH_3$ | H |
| 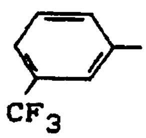 | 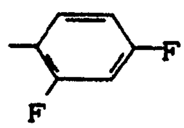 | $CH_3$ | $COCF_3$ |
| 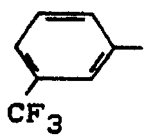 | 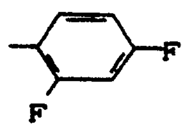 | H | OH |
| 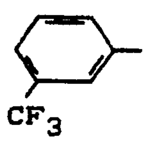 | 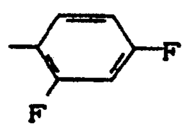 | $CH_3$ | OH |

Tabelle 1 - Fortsetzung

| $R^1$ | $R^2$ | $R^3$ | $R^4$ |
|---|---|---|---|
| 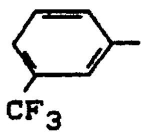 | 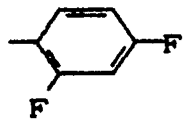 | H | $NHCH_3$ |
| 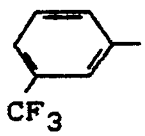 | 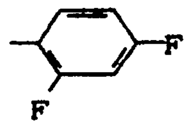 | $CH_3$ | $NH_2$ |
| 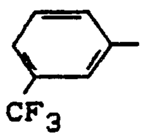 | 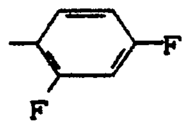 | $CH_3$ | $NHCH_3$ |
| $-CH_2-CH=CHCH_3$ | 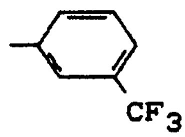 | $CH_3$ | H |
| $-CH_2-CH=CHCH_3$ | 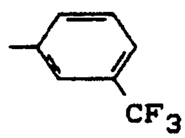 | $CH_3$ | $-NHCH_3$ |
| $-CH_2-CH=CHCH_3$ | 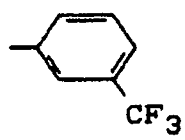 | $CH_3$ | $-COCF_3$ |
| $-CH_2-CH=CHCH_3$ | 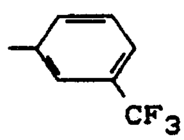 | H | OH |
| $-CH_2-CH=CHCH_3$ | 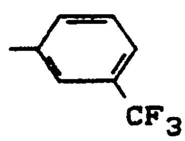 | $CH_3$ | OH |

Tabelle 1 - Fortsetzung

| $R^1$ | $R^2$ | $R^3$ | $R^4$ |
|---|---|---|---|
| $-CH_2-CH=C(CH_3)_2$ | 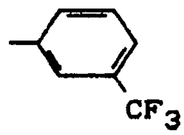 | $CH_3$ | H |
| $-CH_2-CH=C(CH_3)_2$ | 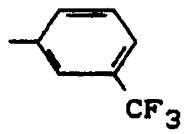 | $CH_3$ | $COCF_3$ |
| 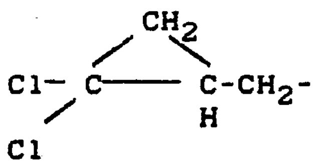 | 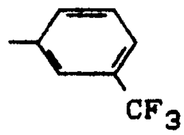 | $CH_3$ | $-NH_2$ |
| 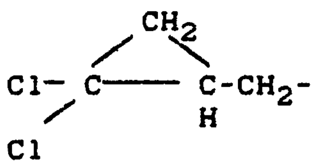 | 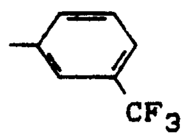 | $CH_3$ | $-NHCH_3$ |
| 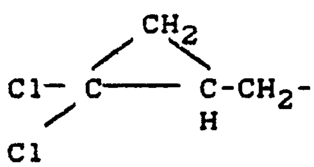 | 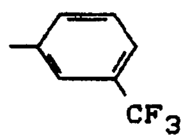 | H | 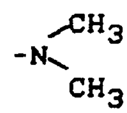 |
| 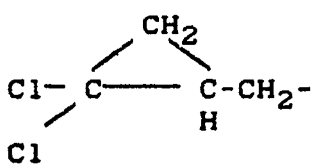 | 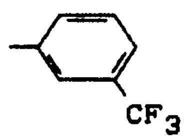 | $COCF_3$ | 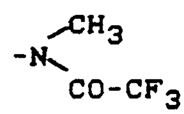 |
| 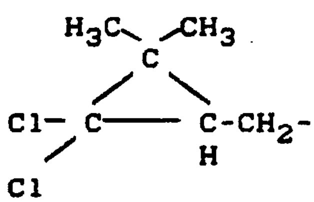 | 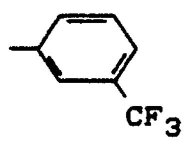 | H | $CH_3$ |

**Tabelle 1** - Fortsetzung

| $R^1$ | $R^2$ | $R^3$ | $R^4$ |
|---|---|---|---|
| 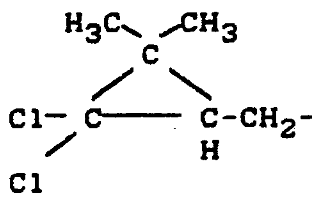 | 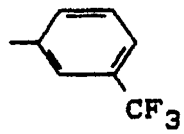 | $CH_3$ | $-NH_2$ |
| 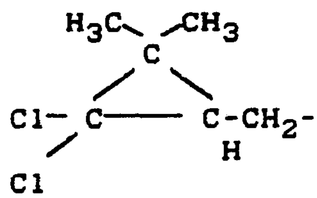 | 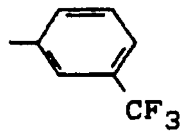 | $CH_3$ | $-NHCH_3$ |
| 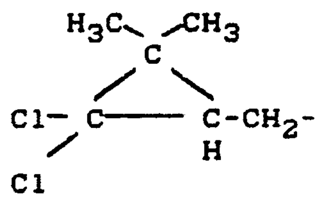 | 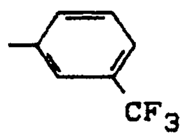 | $-COCF_3$ | 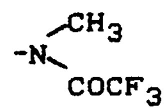 |
| 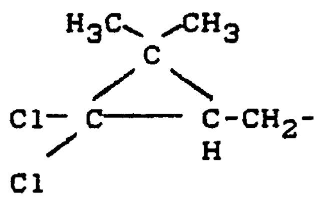 | 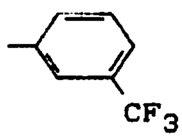 | H | -OH |
| 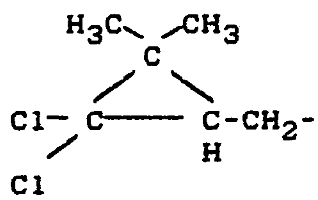 | 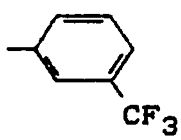 | $CH_3$ | -OH |
| 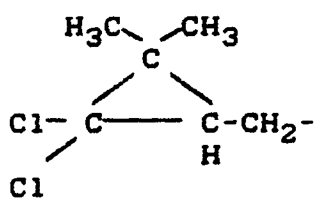 | 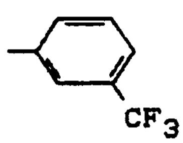 | $CH_3$ | $-OCOCF_3$ |

**Tabelle 1** - Fortsetzung

| $R^1$ | $R^2$ | $R^3$ | $R^4$ |
|---|---|---|---|
| 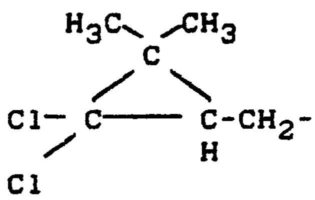 | 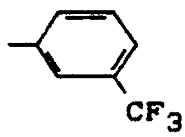 | $CH_3$ | $-COCF_3$ |
| 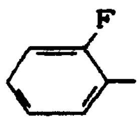 | 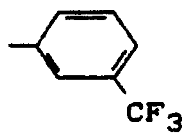 | H | $CH_3$ |
| 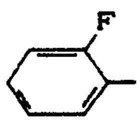 | 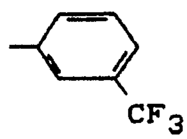 | $COCF_3$ | $CH_3$ |
| 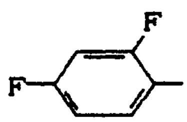 | 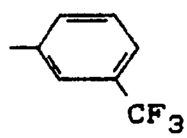 | H | $CH_3$ |
| 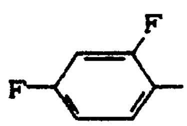 | 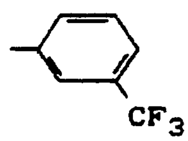 | H | OH |
| 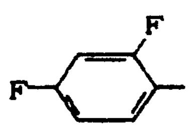 | 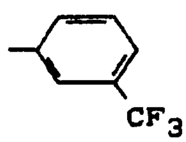 | $CH_3$ | OH |
| 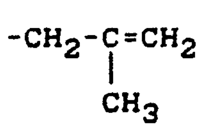 | 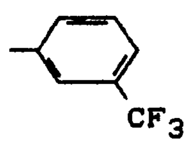 | $CH_3$ | H |

Tabelle 1 - Fortsetzung

| $R^1$ | $R^2$ | $R^3$ | $R^4$ |
|---|---|---|---|
| $-CH_2-C=CH_2$<br>\|<br>$CH_3$ | 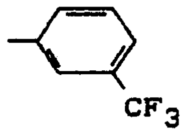 | $CH_3$ | $NHCH_3$ |
| $-CH_2-C=CH_2$<br>\|<br>$CH_3$ | 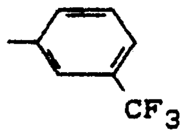 | H | $NHCH_3$ |
| $-CH_2-C=CH_2$<br>\|<br>$CH_3$ | 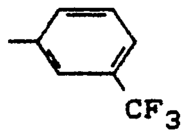 | H | OH |
| $-CH_2-C=CH_2$<br>\|<br>$CH_3$ | 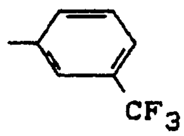 | $-COCH_3$ | $OCOCH_3$ |
| $-CH_2-C=CH_2$<br>\|<br>$CH_3$ | 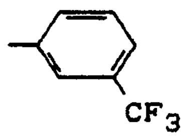 | $-COCF_3$ | $CH_3$<br>$-N$<br>$COCF_3$ |
| $-CH_2-C=CH_2$<br>\|<br>Cl | 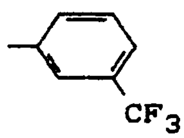 | $CH_3$ | $-NHCH_3$ |
| $-CH_2-C=CH_2$<br>\|<br>Cl | 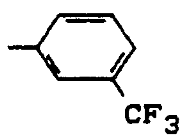 | H | $-NHCH_3$ |
| $-CH_2-C=CH_2$<br>\|<br>Cl | 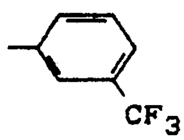 | $CH_3$ | $-NH_2$ |

Tabelle 1 - Fortsetzung

| $R^1$ | $R^2$ | $R^3$ | $R^4$ |
|---|---|---|---|
| 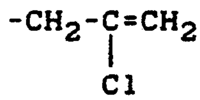 | 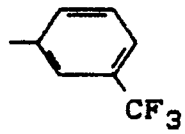 | $CH_3$ | $-COCH_3$ |
| 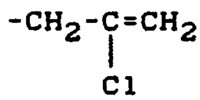 | 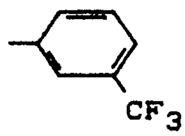 | H | -OH |
| $-CH_2-CH=C(Cl_2)$ | 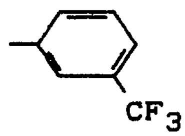 | $CH_3$ | -OH |
| $-CH_2-CH=C(Cl_2)$ | 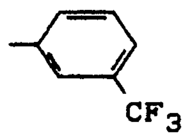 | $CH_3$ | -H |
| $-CH_2-CH=C(Cl_2)$ | 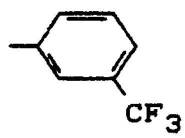 | $CH_3$ | $-NHCH_3$ |
| $-CH_2-CH=C(Cl_2)$ | 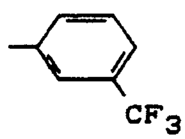 | H | $-NHCH_3$ |
| $-CH_2-CH=C(Cl_2)$ | 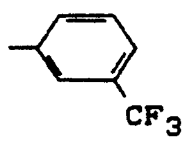 | $-CH_3$ | 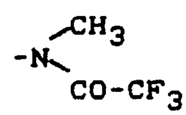 |
| 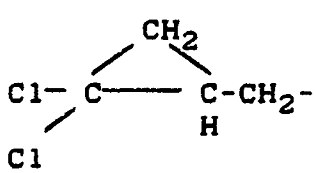 | 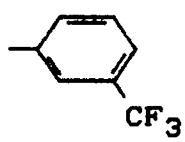 | $CH_3$ | 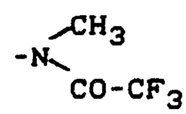 |

Tabelle 1 - Fortsetzung

| $R^1$ | $R^2$ | $R^3$ | $R^4$ |
|---|---|---|---|
| 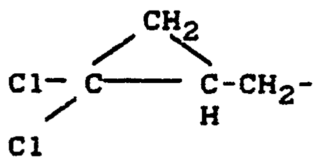 | 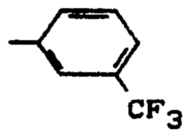 | $CH_3$ | -OH |
| $CH_3OCH_2$- | 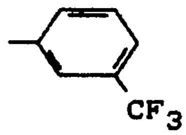 | $CH_3$ | H |
| $CH_3OCH_2$- | 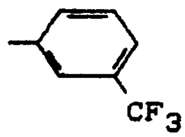 | $CH_3$ | $-COCF_3$ |
| $CH_3OCH_2$- | 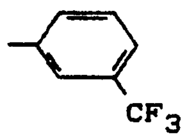 | H | $NHCH_3$ |
| $CH_3OCH_2$- | 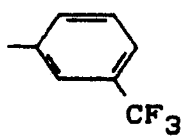 | H | OH |
| $CH_3OCH_2$- | 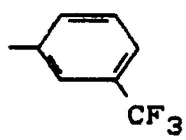 | $CH_3$ | OH |
| 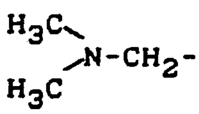 | 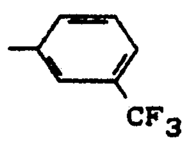 | $CH_3$ | H |
| 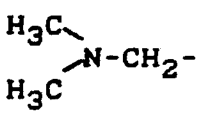 | 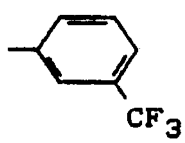 | $CH_3$ | $COCF_3$ |

**Tabelle 1 - Fortsetzung**

| $R^1$ | $R^2$ | $R^3$ | $R^4$ |
|---|---|---|---|
| 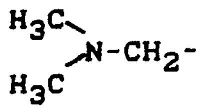 | 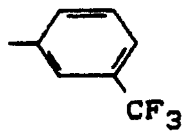 | $CH_3$ | $NHCH_3$ |
| 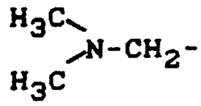 | 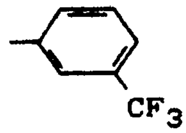 | H | OH |
| 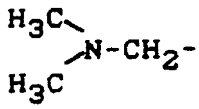 | 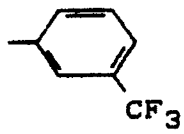 | $CH_3$ | OH |
| 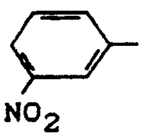 | 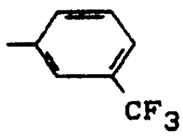 | $CH_3$ | H |
| 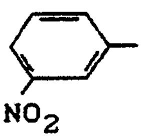 | 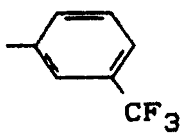 | H | $NHCH_3$ |
| 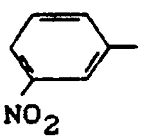 | 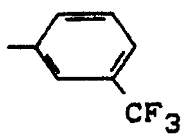 | H | OH |
| 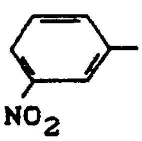 | 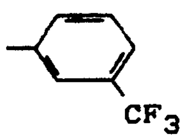 | $CH_3$ | OH |

Verwendet man beispielsweise 2-[4-Fluorphenyl]-4-[3-trifluormethylphenyl]-5-chlor-2H-pyridazin-3-on und Methylamin als Ausgangsstoffe, so läßt sich der Reaktionsablauf des erfindungsgemäßen Verfahrens (a) durch das folgende Formelschema darstellen:

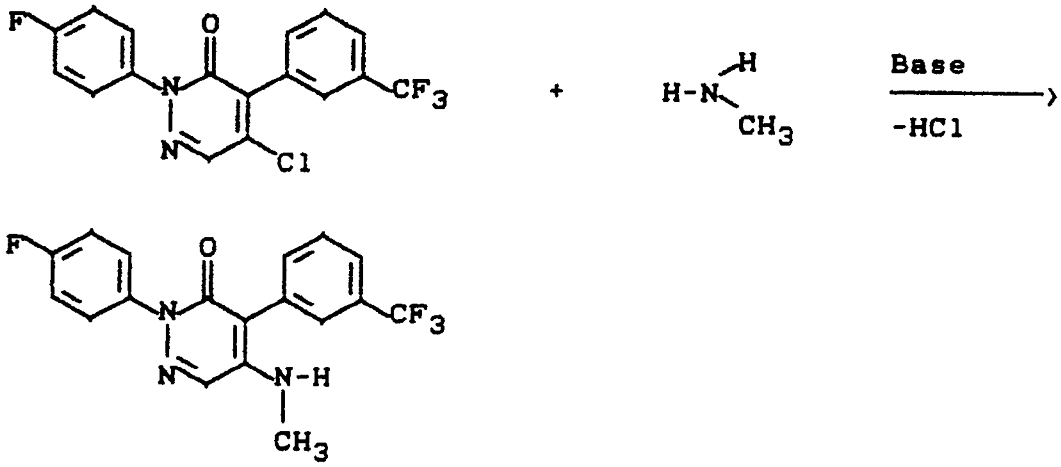


Verwendet man beispielsweise nach Verfahren (a) hergestelltes 2-[4-Fluorphenyl]-4-[3-trifluormethylphenyl]-5-methylamino-2H-pyridazin-3-on und Acetylchlorid als Ausgangsstoffe, so läßt sich der Reaktionsablauf des erfindungsgemäßen Verfahrens (b) durch das folgende Formelschema darstellen:

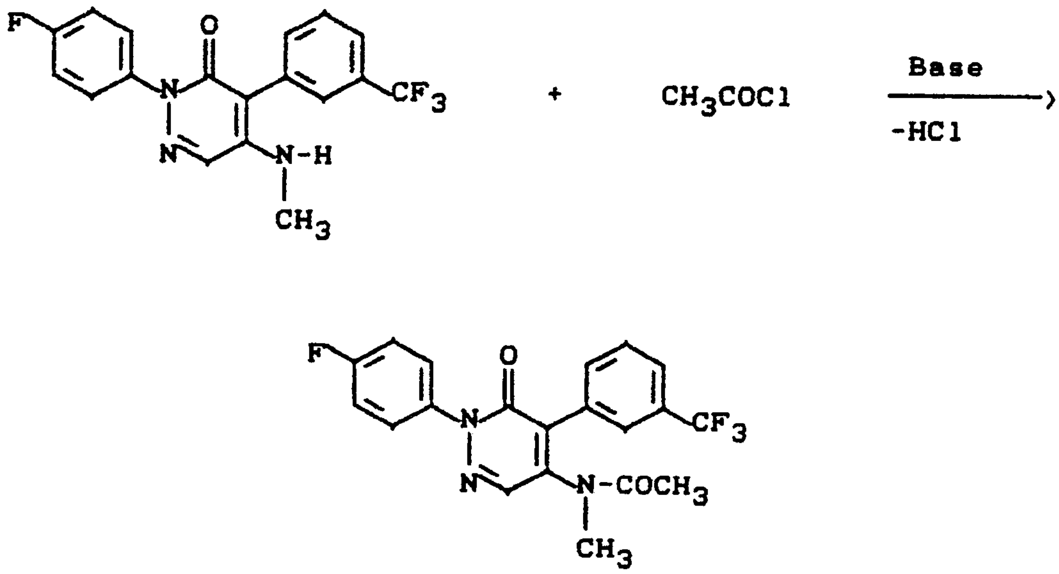


Die beim erfindungsgemäßen Verfahren (a) zur Herstellung von Verbindungen der Formel (I) als Ausgangsstoffe zu verwendenden 2H-5-Halogen-pyridazinon-Derivate sind durch die Formel (II) allgemein definiert.

In Formel (II) haben $R^1$ und $R^2$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$ und $R^2$ angegeben wurden.

X steht in Formel (II) für Halogen, vorzugsweise für Chlor oder Brom, insbesondere für Chlor.

Die 2H-5-Halogen-pyridazinon-Derivate der Formel (II) sind teilweise bekannt und/oder können nach an sich bekannten Verfahren hergestellt werden (vgl. z.B. EP 199281, EP 193853: Latv. PSR Zinat. Akad. Vestis, Kim. Ser., (4), S. 496-497 (1972)).

Noch nicht bekannt sind 2H-5-Halogen-pyridazinon-Derivate der allgemeinen Formel (IIa)

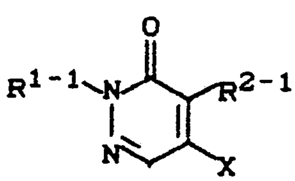

(IIa)

in welcher

$R^{1-1}$ für Alkyl, Alkoxyalkyl, Alkylthioalkyl, Aminoalkyl, (Di)alkylaminoalkyl, Hydroxyalkyl, Halogenalkyl, Alkenyl, Halogenalkenyl, für jeweils gegebenenfalls substituiertes Cycloalkyl, Cycloalkylalkyl, Phenyl oder Phenylalkyl steht;

$R^{2-1}$ für jeweils gegebenenfalls substituiertes Phenyl oder Phenylalkyl steht und

X für Halogen steht.

Bevorzugt sind Verbindungen der Formel (IIa), bei welchen

$R^{1-1}$ für jeweils geradkettiges oder verzweigtes Alkyl, Alkoxyalkyl und (Di)alkylaminoalkyl mit 1 bis 4 Kohlenstoffatomen in den jeweiligen Alkylteilen, für jeweils gegebenenfalls einfach bis dreifach gleich oder verschieden substituiertes Cyclopentyl oder Cyclohexyl steht, wobei als Substituenten Methyl oder Ethyl in Frage kommen, oder für gegebenenfalls einfach bis vierfach gleich oder verschieden substituiertes Cyclopropylmethyl steht, wobei als Substituenten Methyl oder Halogen infrage kommen, für gegebenenfalls durch ein bis vier Halogenatome substituiertes Alkenyl mit 2-6 Kohlenstoff atomen oder für gegebenenfalls einfach bis dreifach gleich oder verschieden im Phenylteil substituiertes Phenyl oder Phenylalkyl mit gegebenenfalls 1 bis 3 Kohlenstoffatomen im geradkettigen oder verzweigten Alkylteil steht, wobei als Substituenten jeweils in Frage kommen:

Halogen, Cyano, Nitro, jeweils geradkettiges oder verzweigtes Alkyl oder Alkoxy mit jeweils 1 bis 6 Kohlenstoffatomen, Halogenalkyl oder Halogenalkoxy mit jeweils 1 oder 2 Kohlenstoffatomen und 1 bis gleichen oder verschiedenen Halogenatomen;

$R^{2-1}$ für gegebenenfalls einfach bis dreifach gleich oder verschieden im Phenylteil substituiertes Phenyl oder Phenylalkyl mit gegebenenfalls 1-3 Kohlenstoffatomen im geradkettigen oder verzweigten Alkylteil steht, wobei als Substituenten jeweils infrage kommen:

Halogen, Cyano, Nitro, jeweils geradkettiges oder verzweigtes Alkyl oder Alkoxy mit jeweils 1-6 Kohlenstoffatomen, Halogenalkyl oder Halogenalkoxy mit jeweils 1 oder 2 Kohlenstoffatomen und 1 bis 5 gleichen oder verschiedenen Halogenatomen, und

X für Chlor oder Brom steht.

Besonders bevorzugt sind Verbindungen der Formel (IIa), bei welchen

$R^{1-1}$ für Methyl, Ethyl, n- oder i-Propyl, n-, i-, s-oder t-Butyl, Methoxymethyl, Ethoxymethyl, Methoxyethyl, Dimethylaminomethyl, Allyl, n- oder i-Bute nyl n- oder i-Pentenyl, 2-Fluorpropen-3-yl, 2-Chlor-propen-3-yl, 1-Chlorpropen-3-yl, 1,1-Dichlorpropen-3-yl, 1-Fluorpropen-3-yl, 1,1-Difluorpropen-3-yl, 1,2-Dichlorpropen-3-yl, 1,2-Difluorpropen-3-yl, 1,1,2-Trichlorpropen-3-yl, Cyclopropylmethyl, 1,1-Dichlorcyclopropylmethyl, 1,1-Difluorcyclopropylmethyl, 1,1-Dimethylcyclopropylmethyl, 1,1-Dimethyl-2,2-dichlorcyclopropylmethyl, für jeweils gegebenenfalls einfach oder zweifach substituiertes Cyclopentyl oder Cyclohexyl steht, wobei als Substituenten Methyl und/oder Ethyl infrage kommen, oder für gegebenenfalls einfach oder zweifach gleich oder verschieden substituiertes Phenyl steht, wobei als Substituenten infrage kommen: Fluor, Chlor, Brom, Methyl, Ethyl, Methoxy, Ethoxy, Trifluormethyl oder Trifluormethoxy,

$R^{2-1}$ für gegebenenfalls einfach oder zweifach gleich oder verschieden substituiertes Phenyl steht, wobei als Substituenten infrage kommen: Fluor, Chlor, Brom, Methyl, Ethyl, Methoxy, Ethoxy, Trifluormethyl oder Trifluormethoxy und

X für Chlor steht.

Man erhält diese neuen Verbindungen der Formel (IIa),

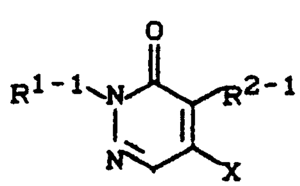

(IIa)

in welcher

$R^{1-1}$, $R^{2-1}$ und X die oben bei Formel (IIa) angegebenen Bedeutungen haben,

wenn man im ersten Reaktionsschritt Hydrazin-Derivate der Formel (V)

$R^{1-1}$-NH-$NH_2$ (V)

in welcher

$R^{1-1}$ die oben angegebene Bedeutung hat, mit Mucohalogensäuren der Formel (VI)

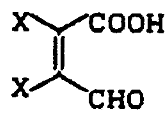

(VI)

in welcher
X für Halogen steht,
gegebenenfalls in Gegenwart eines Verdünnungsmittels wie z.B. Methanol, Ethanol, Toluol oder Essigsäure bei Temperaturen zwischen 0° C und 15° C, vorzugsweise zwischen 20° C und 110° C, umsetzt (vgl. auch Angew. Chem. 77, 282 (1965)) und im zweiten Reaktionsschritt die so erhaltenen 4,5-Dihalogenpyridazinon-Derivate der Formel (VII)

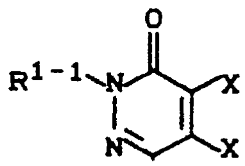

(VII)

in welcher
$R^{1-1}$ die oben angegebene Bedeutung hat und
X für Halogen steht,
mit Grignardverbindungen der allgemeinen Formel (VIII)
$R^{2-1}$-$MgX^1$ (VIII)
in welcher
$R^{2-1}$ die oben angegebene Bedeutung hat und
$X^1$ für Halogen, insbesondere für Chlor oder Brom steht,
gegebenenfalls in Gegenwart eines Verdünnungsmittels wie z.B. Diethylether oder Tetrahydrofuran bei Temperaturen zwischen 0° C und 150° C, vorzugsweise zwischen 20° C und 80° C, umsetzt.

Im Fall von funktionellen Gruppen (Hydroxy, Amino) im Substituenten $R^{1-1}$ sollten diese gegebenenfalls vor Durchführung der Grignard-Reaktion in üblicher Weise durch Schutzgruppen geschützt werden.

Hydrazin-Derivate der Formel (V), Mucohalogensäuren der Formel (VI) und Grignardverbindungen der Formel (VIII) sind allgemein bekannte Verbindungen der organischen Chemie und können in bekannter Art und Weise erhalten werden.

Die als Zwischenprodukte entstehenden Verbindungen der Formel (VII) sind bekannt bzw. können in bekannter Art und Weise hergestellt werden, wie z.B. auch durch Alkylierung der entsprechenden unsubstituierten 4,5-Dihalogen-3(2H)-pyridazinon-Derivate der Formel (VII) (vgl. z.B. Monatshefte für Chemie 99, 15-84 (1968), Synth. Commun. 1981, 631).

Die außerdem beim erfindungsgemäßen Verfahren (a) zur Herstellung von Verbindungen der Formel (I) als Ausgangsstoffe zu verwendenden Amine sind durch die Formel (III) allgemein definiert.

In Formel (III) haben $R^3$ und $R^4$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^3$ und $R^4$ angegeben wurden.

Amine der Formel (III) sind in der organischen Chemie allgemein bekannte Verbindungen.

Die beim erfindungsgemäßen Verfahren (b) zur Herstellung von Verbindungen der Formel (I) als Ausgangsstoffe zu verwendenden 2H-Pyridazinon-Derivate sind durch die Formel (Ia) allgemein definiert.

In Formel (Ia) haben $R^1$, $R^2$ und $R^3$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$ und $R^3$ angegeben wurden $R^{4-1}$ steht vorzugsweise für Wasserstoff, Hydroxy, Amino oder für $C_1$-$C_4$-Alkylamino (insbesondere Methylamino).

2H-Pyridazinon-Derivate der Formel (Ia) sind erfindungsgemäße Verbindungen.

Die beim erfindungsgemäßen Verfahren (b) zur Herstellung von Verbindungen der Formel (I) als Ausgangsstoffe zu verwendenden Acylierungsmittel sind durch die Formel (IV) allgemein definiert.

In Formel (IV) steht
$R^{4-2}$ vorzugsweise für geradkettiges oder verzweigtes (Di)Alkylaminocarbonyl mit 1-4 Kohlenstoffatomen in den jeweiligen Alkylteilen, jeweils geradkettiges oder verzweigtes Alkoxycarbonyl oder Alkoxycarbonylcarbonyl mit 1-4 Kohlenstoffatomen im Al kylteil, sowie für geradkettiges oder verzweigtes und gegebenenfalls

durch 1-3 Halogenatome substituiertes Alkylcarbonyl mit 1-4 Kohlenstoffatomen im Alkylteil;
insbesondere steht

$R^{4-2}$ für Methylaminocarbonyl, Dimethylaminocarbonyl, Ethylaminocarbonyl, Diethylaminocarbonyl oder Methylethylaminocarbonyl, für Methoxycarbonyl oder Ethoxycarbonyl, Methoxycarbonylcarbonyl oder Ethoxycarbonylcarbonyl sowie für Formyl, Acetyl, Propionyl, Trifluoracetyl, Chloracetyl, Dichloracetyl oder Trichloracetyl.

Z steht in Formel (IV) vorzugsweise für Halogen, insbesondere für Chlor oder Brom.

In einer besonderen Ausführungsform des erfindungsgemäßen Verfahrens (b) können als Acylierungsmittel der Formel (IV) auch entsprechende Carbonsäureanhydride eingesetzt werden.

Als Verdünnungsmittel zur Durchführung der erfindungsgemäßen Verfahren (a) und (b) kommen inerte organische Lösungsmittel oder wäßrige Systeme infrage. Zu den organischen Lösungsmitteln gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Als Säurebindemittel zur Durchführung der erfindungsgemäßen Verfahren (a) und (b) kommen alle üblicherweise verwendbaren anorganischen und organischen Basen infrage. Vorzugsweise verwendet man Alkalimetallhydride, -hydroxide, -amide, -carbonate oder -hydrogencarbonate, wie beispielsweise Natriumhydrid, Natriumamid, Natriumhydroxid, Natriumcarbonat oder Natriumhydrogencarbonat oder auch tertiäre Amine, wie beispielsweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Bei dem erfindungsgemäßen Verfahren (a) ist es auch möglich, die als Reaktionsteilnehmer verwendeten Amine der Formeln (III) in entsprechendem Überschuß gleichzeitig als Säurebindemittel einzusetzen.

Die Reaktionstemperaturen können bei der Durchführung der erfindungsgemäßen Verfahren (a) und (b) in einem größeren Bereich variiert werden. Im allgemeinen arbei tet man bei Temperturen zwischen -20 °C und +150 °C, vorzugsweise bei Temperaturen zwischen 0 °C und +120 °C.

Die erfindungsgemäßen Verfahren (a) und (b) werden üblicherweise unter Normaldruck durchgeführt. Es ist jedoch auch möglich unter erhöhtem Druck zu arbeiten.

Zur Durchführung des erfindungsgemäßen Verfahrens (a) setzt man pro Mol an 2H-5-Halogenpyridazinon-Derivat der Formel (II) im allgemeinen 1,0 bis 10,0 Mol, vorzugsweise 1,0 bis 5,0 Mol, an Amin der Formel (III) und gegebenenfalls 1,0 bis 10,0 Mol, vorzugsweise 1,0 5,0 Mol, an Säurebindemittel ein.

Zur Durchführung des erfindungsgemäßen Verfahrens (b) setzt man pro Mol an 2H-Pyridazinon-Derivat der Formel (Ia) im allgemeinen 1,0 bis 5,0 Mol, vorzugsweise 1,0 bis 2,0 Mol, an Acylierungsmittel der Formel (IV) und 1,0 bis 5,0 Mol, vorzugsweise 1,0 bis 2,0 Mol, an Säurebindemittel ein.

Die Reaktionsdurchführung, Aufarbeitung und Isolierung der Reaktionsprodukte der Formel (I) erfolgt in beiden Fällen nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind.

Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Dabei eignen sich die erfindungsgemäßen Wirkstoffe besonders gut zur selektiven Bekämpfung dikotyler und monokotyler Unkräuter in monokotylen und dikotylen Kulturen im Vorauflauf- und Nachauflaufverfahren.

Die erfindungsgemäßen Wirkstoffe greifen ferner in den Metabolismus der Pflanzen ein und können deshalb zum Teil auch als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung, bezogen auf das Entwicklungsstadium der Pflanze, sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Unter dem Einfluß von Wachstumsregulatoren kann der Blattbestand der Pflanzen so gesteuert werden, daß ein Entblättern der Pflanzen zu einem gewunschten Zeitpunkt erreicht wird. Eine derartige Entlaubung spielt bei der mechanischen Beerntung der Baumwolle eine große Rolle, ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Ta bakstengeln; als Emulgier- und/oder schaumerzeugende Mittel kommen infrage: z.B. nichtionogene und anionische Emulgatoren, wie Polyoxyethylen-Fettsäuren-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können als solche oder in ihren Formulierungen auch in Mischung

mit bekannten Herbiziden zur Unkrautbekämpfung Verwendung finden, wo bei Fertigformulierungen oder Tankmischungen möglich sind.

Für die Mischungen kömmen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N′-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen infrage.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,001 und 10 kg Wirkstoff pro Hektar Bodenfläche, vorzugsweise zwischen 0,005 und 5 kg pro ha.

Die Herstellung und die Verwendung der erfindungsgemäßen Wirkstoffe geht aus den nachfolgenden Beispielen hervor.

Herstellungsbeispiele

Beispiel 1

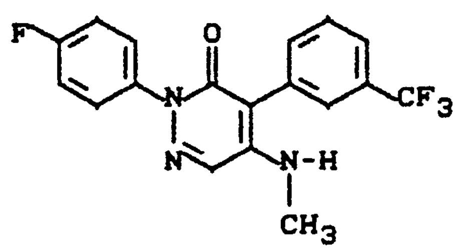

(Verfahren (a))

Eine Mischung aus 1,4 g (0,0038 Mol) 2-[4-Fluorphenyl]-4-[3-trifluormethylphenyl]-5-chlor-2H-pyridazin-3-on und 1,2 g (0,039 Mol) Methylamin (30%ige Lösung in $H_2O$) in 50 ml Ethanol wird 16 Stunden bei Raumtemperatur gerührt. Anschließend wird die Reaktionsmischung eingeengt, der Rückstand mit Methylenchlorid/Wasser aufgenommen. Die organische Phase wird über Natriumsulfat getrockent, eingeengt und an Kieselgel 60 mit Methylenchlorid chromatographiert.

Man erhält 0,5 g (35% der Theorie) an 2-[4-Fluorphenyl]-4-[3-trifluormethylphenyl]-5-methylamino-2H-pyridazin-3-on mit dem Schmelzpunkt 161-162° C.

In entsprechender Weise und gemäß den allgemeinen Angaben zu den Verfahren (a) und (b) erhält man die folgenden 2H-Pyridazinon-Derivate der allgemeinen Formel (I)

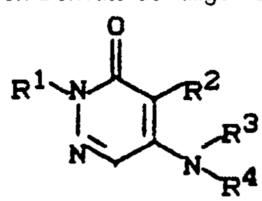

(I)

<u>**Tabelle 2**</u>

| Bsp.-Nr. | $R^1$ | $R^2$ | $R^3$ | $R^4$ | Schmelzpunkt bzw. Brechungsindex |
|---|---|---|---|---|---|
| 2 | 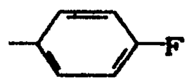 | 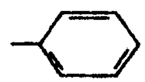 | H | $-CH_3$ | 174 - 175° C |
| 3 | 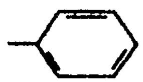 | 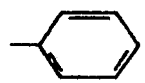 | H | $-CH_3$ | 156 - 157° C |
| 4 | 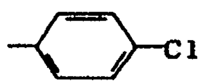 | 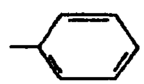 | H | $-CH_3$ | 204 - 205° C |
| 5 | 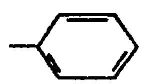 | 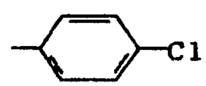 | H | $-CH_3$ | 58 - 60° C |
| 6 | 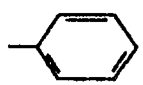 | 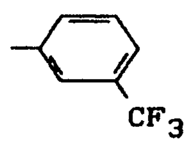 | H | $-CH_3$ | 58 - 60° C |
| 7 | $-CH_3$ | 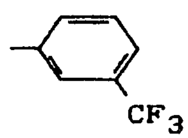 | H | $-CH_3$ | 117 - 118° C |

**Tabelle 2** - Fortsetzung

| Bsp.-Nr. | $R^1$ | $R^2$ | $R^3$ | $R^4$ | Schmelzpunkt bzw. Brechungsindex |
|---|---|---|---|---|---|
| 8 | 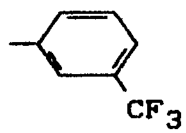 | 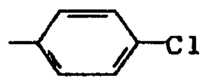 | H | $-CH_3$ | 115° C |
| 9 | 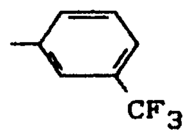 | 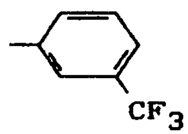 | H | $-CH_3$ | 99 - 100° C |
| 10 | $-CH_2-$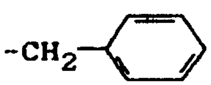 | 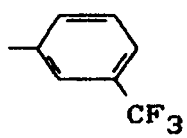 | H | $-CH_3$ | $n_D^{20}$ = 1,5838 |
| 11 | 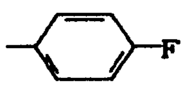 | 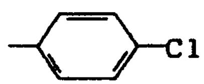 | H | $-CH_3$ | 177 - 178° C |
| 12 | 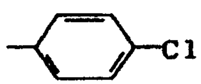 | 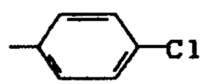 | H | $-CH_3$ | 185 - 186° C |
| 13 | $-CH_3$ | 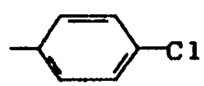 | H | $-CH_3$ | 58 - 60° C |

**Tabelle 2** - Fortsetzung

| Bsp.-Nr. | $R^1$ | $R^2$ | $R^3$ | $R^4$ | Schmelzpunkt bzw. Brechungs-index |
|---|---|---|---|---|---|
| 14 | $-CH_2-$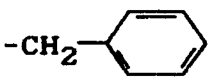 | 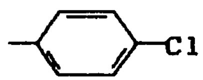 | H | $-CH_3$ | 84 - 85° C |
| 15 | 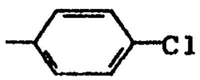 | 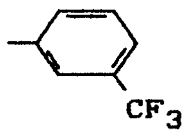 | H | $-CH_3$ | 75 - 76° C |
| 16 | 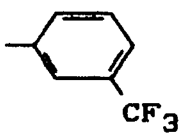 | 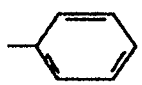 | H | $-CH_3$ | 147 - 148° C |
| 17 | 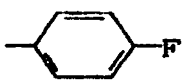 | 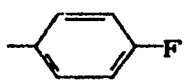 | H | $-CH_3$ | 81 - 83° C |
| 18 | 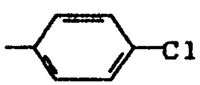 | 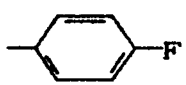 | H | $-CH_3$ | 81 - 83° C |
| 19 | 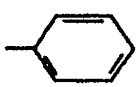 | 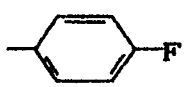 | H | $-CH_3$ | 62 - 64° C |

Tabelle 2 - Fortsetzung

| Bsp.-Nr. | $R^1$ | $R^2$ | $R^3$ | $R^4$ | Schmelzpunkt bzw. Brechungsindex |
|---|---|---|---|---|---|
| 20 | 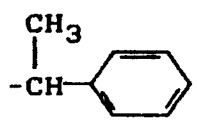 | 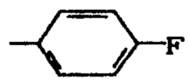 | H | $-CH_3$ | 138 - 139° C |
| 21 | 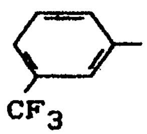 | 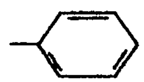 | H | H | 208° C |
| 22 | 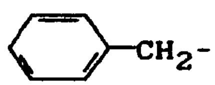 | 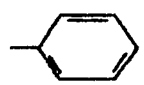 | H | $-CH_3$ | 146° C |
| 23 | 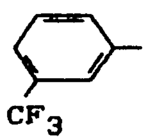 | 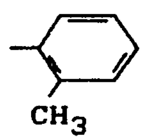 | H | $-CH_3$ | 148° C |
| 24 | 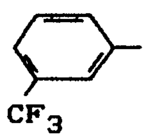 | 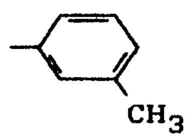 | H | $-CH_3$ | 64 - 67° C |

<u>Tabelle 2</u> - Fortsetzung

| Bsp.-Nr. | $R^1$ | $R^2$ | $R^3$ | $R^4$ | Schmelzpunkt bzw. Brechungsindex |
|---|---|---|---|---|---|
| 25 | 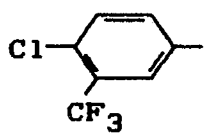 | 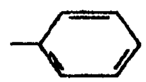 | H | $-CH_3$ | 62 - 64° C |
| 26 | 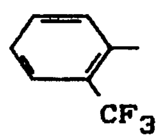 | 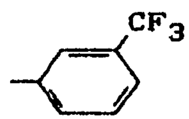 | H | $-CH_3$ | 95 - 97° C |
| 27 | 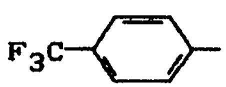 | 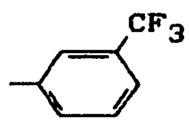 | H | $-CH_3$ | 148 - 149° C |
| 28 | 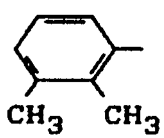 | 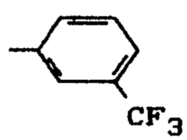 | H | $CH_3$ | 74° C |
| 29 | $C_2H_5$- | 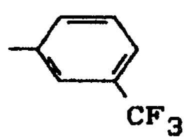 | H | $CH_3$ | 144 - 149° C |

**Tabelle 2** - Fortsetzung

| Bsp.-Nr. | $R^1$ | $R^2$ | $R^3$ | $R^4$ | Schmelzpunkt bzw. Brechungsindex |
|---|---|---|---|---|---|
| 30 | $n-C_3H_7-$ | 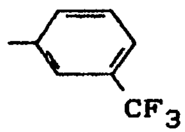 | H | $CH_3$ | 94 - 97° C |
| 31 | $n-C_4H_9-$ | 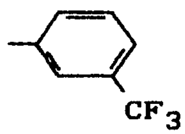 | H | $CH_3$ | $n_D^{20}$ = 1.5467 |
| 32 | 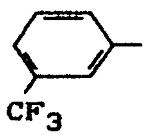 | 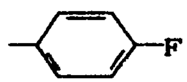 | H | $CH_3$ | 158° C |
| 33 | 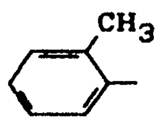 | 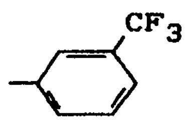 | H | $CH_3$ | 91 - 93° C |
| 34 | 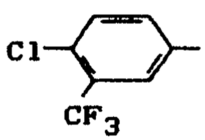 | 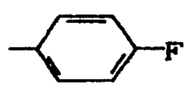 | H | $CH_3$ | 74 - 75° C |

Tabelle 2 - Fortsetzung

| Bsp.-Nr. | $R^1$ | $R^2$ | $R^3$ | $R^4$ | Schmelzpunkt bzw. Brechungsindex |
|---|---|---|---|---|---|
| 35 | 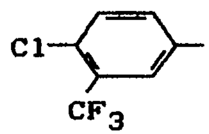 | 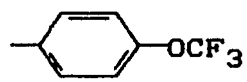 | H | $CH_3$ | 64 - 65° C |
| 36 | 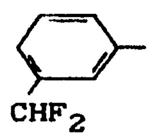 | 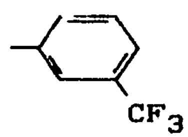 | H | $CH_3$ | 45 - 47° C |
| 37 | $CH_2=CH-CH_2-CH_2$ | 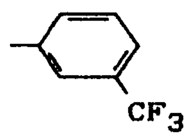 | H | $CH_3$ | 61 - 62° C |
| 38 | 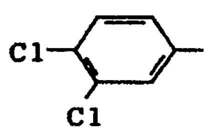 | 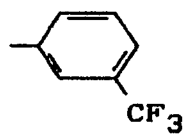 | H | $CH_3$ | 51 - 52° C |
| 39 | 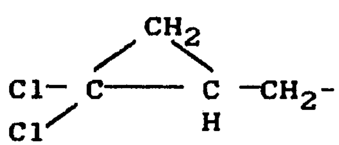 | 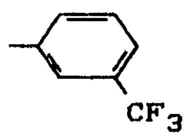 | H | $CH_3$ | $n_D^{20}$ = 1.5236 |

<u>Tabelle 2</u> - Fortsetzung

| Bsp.-Nr. | $R^1$ | $R^2$ | $R^3$ | $R^4$ | Schmelzpunkt bzw. Brechungsindex |
|---|---|---|---|---|---|
| 40 | 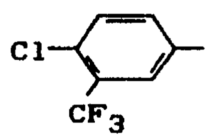 | 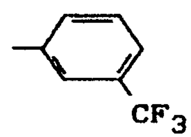 | H | $CH_3$ | 75 - 76° C |
| 41 | 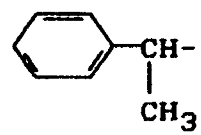 | 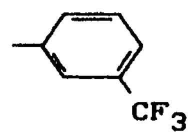 | H | $CH_3$ | amorph |
| 42 | 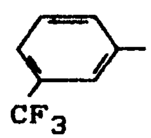 | 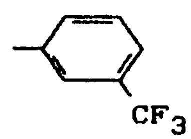 | $CH_3$ | $CH_3$ | 110 - 111° C |
| 43 | 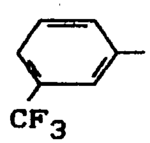 | 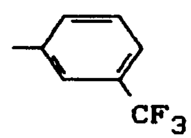 | H | $-CH_2-CH_2-OCH_3$ | $n_D^{20}$ = 1.5278 |
| 44 | 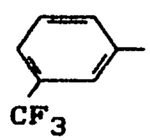 | 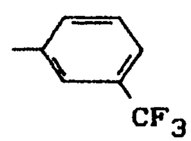 | H | $n-C_3H_7$ | 95 - 96° C |

**Tabelle 2** - Fortsetzung

| Bsp.-Nr. | $R^1$ | $R^2$ | $R^3$ | $R^4$ | Schmelzpunkt bzw. Brechungsindex |
|---|---|---|---|---|---|
| 45 | 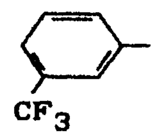 | 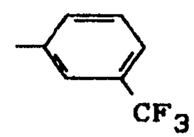 | $CH_3$ | $-NH_2$ | 88° C |
| 46 | 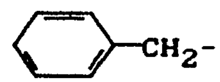 | 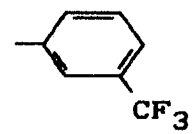 | $CH_3$ | $-COCH_3$ | amorph |
| 47 | 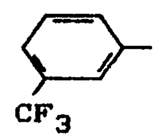 | 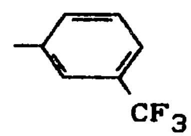 | H | H | 68 - 70° C |
| 48 | 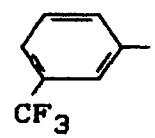 | 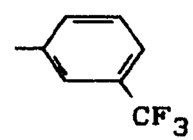 | H | $-OCH_3$ | 42 - 45° C |
| 49 | 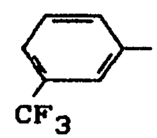 | 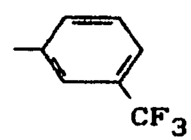 | $CH_3$ | -OH | zähes Öl |

<u>Tabelle 2</u> - Fortsetzung

| Bsp.-Nr. | $R^1$ | $R^2$ | $R^3$ | $R^4$ | Schmelzpunkt bzw. Brechungs-index |
|---|---|---|---|---|---|
| 50 | 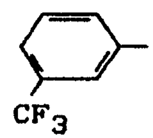 | 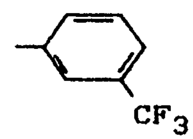 | $CH_3$ | $-COCF_3$ | 122° C |
| 51 | 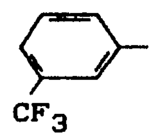 | 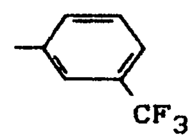 | H | $-COCH_3$ | 178 - 180° C |
| 52 | 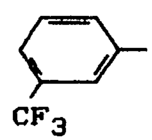 | 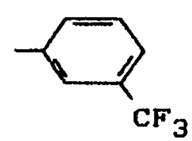 | $-COCH_3$ | $-COCH_3$ | 174° C |
| 53 | 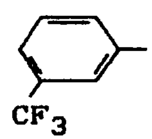 | 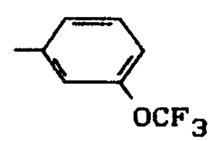 | H | $-CH_3$ | 47 - 51° C |
| 54 | $CH_2=C-CH_2-$<br>$CH_3$ | 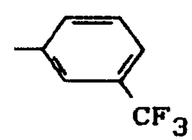 | H | $-CH_3$ | 92° C |

<u>Tabelle 2</u> - Fortsetzung

| Bsp.-Nr. | $R^1$ | $R^2$ | $R^3$ | $R^4$ | Schmelzpunkt bzw. Brechungs-index |
|---|---|---|---|---|---|
| 55 | 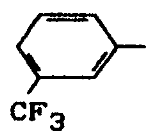 | 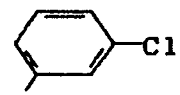 | H | $-CH_3$ | zähes Öl |
| 56 | 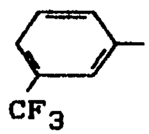 | 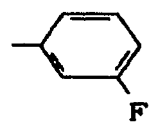 | H | $-CH_3$ | 68 - 70° C |
| 57 | 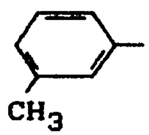 | 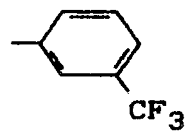 | H | $-CH_3$ | 35 - 38° C |
| 58 | $Cl^{\sim}CH=CH-CH_2-$ | 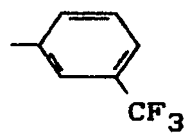 | H | $-CH_3$ | zähes Öl |
| 59 | 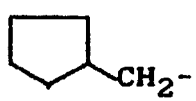 | 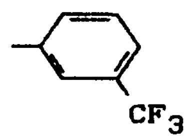 | H | $-CH_3$ | zähes Öl |

<u>Tabelle 2</u> - Fortsetzung

| Bsp.-Nr. | $R^1$ | $R^2$ | $R^3$ | $R^4$ | Schmelzpunkt bzw. Brechungsindex |
|---|---|---|---|---|---|
| 60 | $CH_2=C(F)-CH_2-$ | 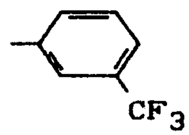 | H | $-CH_3$ | zähes Öl |
| 61 | 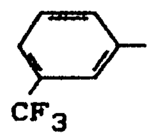 | 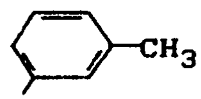 | $-CH_3$ | $-COCH_3$ | 148° C |
| 62 | 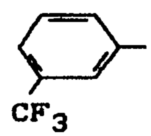 | 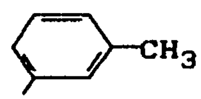 | $-CH_3$ | $-COCF_3$ | 132° C |
| 63 | $n-C_3H_7$ | 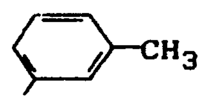 | $-CH_3$ | $-COCF_3$ | zähes Öl |
| 64 | $CH_2=CH-CH_2-$ | 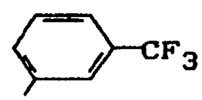 | $-CH_3$ | $-COCF_3$ | zähes Öl |

**Tabelle 2 - Fortsetzung**

| Bsp.-Nr. | $R^1$ | $R^2$ | $R^3$ | $R^4$ | Schmelzpunkt bzw. Brechungsindex |
|---|---|---|---|---|---|
| 65 | 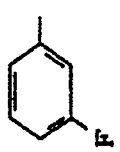 | 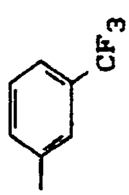 | H | $CH_3$ | 46° C |
| 66 | 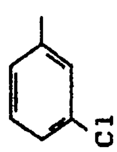 | 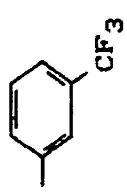 | H | $CH_3$ | 80° C |
| 67 | 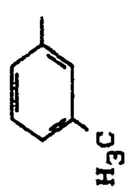 | 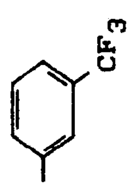 | $CH_3$ | $NH_2$ | 123° C |
| 68 | 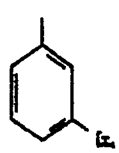 | 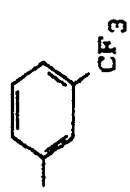 | $CH_3$ | $NH_2$ | 104° C |
| 69 | 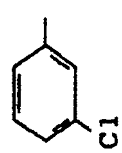 | 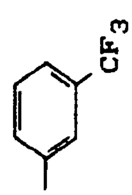 | $CH_3$ | $NH_2$ | 82° C |
| 70 | 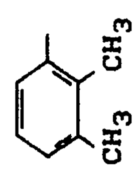 | 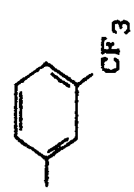 | $CH_3$ | $NH_2$ | 95° C |

Die in Tabelle 2 als Beispiel 39 aufgeführte Verbindung kann beispielsweise wie folgt hergestellt werden:

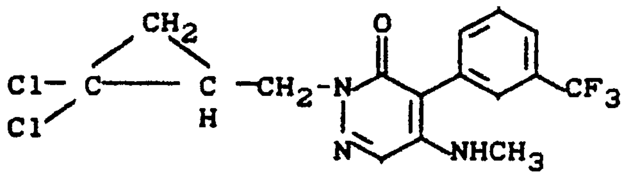

Zu einer Grignard-Lösung, hergestellt aus 1 g (0,04 Mol) Magnesium und 9 g (0,04 Mol) 3-Trifluormethyl-brombenzol in 80 ml Diethylether, fügt man 100 ml absolutes Toluol hinzu, erwärmt auf 60 °C und tropft dann eine Lösung von 5,8 g (0,02 Mol) 2-[1,1-Dichlorcyclopropyl-2-methyl]-4,5-dichlor-2H-pyridazin-3-on, gelöst in 100 ml absolutem Toluol, und rührt die Reaktionsmischung 3 Stunden bei 60 °C. Anschließend hydrolysiert man mit einer Mischung aus Eis und 1N-Salzsäure. Die Etherphase wird abgetrennt, mit Wasser gewaschen, über Natriumsulfat getrocknet und eingedampft. Ohne weitere Reinigung wird der zurückbleibende Sirup für die weitere Umsetzung verwendet.

Das Rohprodukt der Grignard-Reaktion wird in 50 ml Ethanol gelöst, mit 10 g (0,1 Mol) einer 30%igen Lösung von Methylamin in Wasser versetzt und 16 Stunden unter Rückfluß erhitzt. Anschließend wird die Reaktionsmischung eingeengt, der Rückstand mit Methylenchlorid/Wasser aufgenommen, die organische Phase über Natrium sulfat getrocknet und eingedampft. Nach Chromatographie des Rückstandes über Kieselgel 60 mit Methylenchlorid/Methanol (80:1) erhält man 3,4 g (43% d.Th.) von 2-[1,1-Dichlorcyclopropyl-2-methyl]-4-[3-trifluormethylphenyl]-5-methylamino-2H-pyridazin-3-on mit dem Brechungsindex $n_D^{20}$ :1.5236.

Die in Tabelle 2 als Beispiel 50 aufgeführte Verbindung kann beispielsweise wie folgt hergestellt werden:

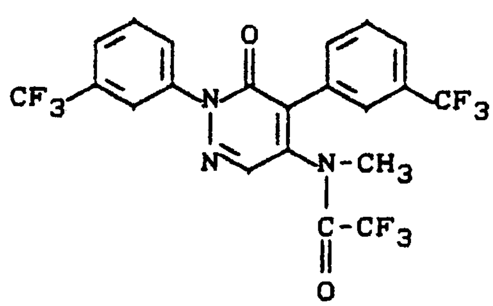

Zu einer Lösung von 4,1 g (0,01 Mol) 2,4-Di-[3-trifluormethylphenyl]-5-methylamino-2H-pyridazin-3-on in 50 ml Pyridin tropft man bei 10 °C 4,2 g (0,02 Mol) Trifluoracetanhydrid und rührt anschließend 16 Stunden bei Raumtemperatur. Die Reaktionsmischung wird mit Methylenchlorid verdünnt und mit Wasser und dreimal mit 2H-Salzsäure extrahiert. Die organische Phase wird mit Natriumsulfat getrocknet und eingedampft. Nach Chromatographie des Rückstandes an Kieselgel 60 mit Methylenchlorid erhält man 3,5 g (68% d.Th.) an 2,4-Di-[3-Trifluormethylphenyl]-5-[trifluoracetyl-methylamino]-2H-pyridazin-3-on vom Schmelzpunkt 122 °C.

Die in Tabelle 2 als Beispiel 47 aufgeführte Verbindung kann beispielsweise wie folgt hergestellt werden:

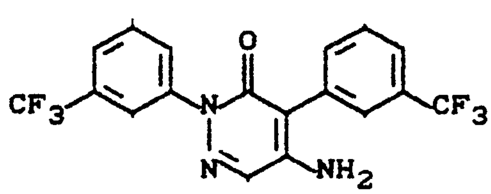

Eine Mischung aus 21,9 g (0,05 Mol) 2,4-Di-[3-trifluormethylphenyl]-5-chlor-2H-pyridazin-3-on und 100 ml Ethanol wird in Gegenwart von überschüssigem Ammoniak (1 Mol) 6 Stunden bei 15 °C im Autoklaven umgesetzt. Die Reaktionsmischung wird eingedampft und der Rückstand über Kieselgel 60 mit Methylenchlorid chromatographiert.

Man erhält 18 g (90% d.Th.) an 2,4-Di-[3-trifluormethylphenyl]-5-amino-2H-pyridazin-3-on vom Schmelzpunkt 68-70 °C.

Die in Tabelle 2 als Beispiel 52 aufgeführte Verbindung kann beispielsweise wie folgt hergestellt werden:

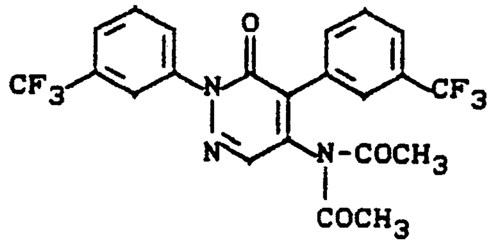

Zu einer Lösung von 4 g (0,01 Mol) 2,4-Di-[3-Trifluormethylphenyl]-5-amino-2H-pyridazin-3-on in 50 ml Dioxan gibt man 0,62 g (0,02 Mol) NaH (80% Dispersion) und erwärmt 30 Minuten auf 40 °C. Danach kühlt man ab, fügt tropfenweise bei 15-20 °C 1,6 g (0,02 Mol) Acetylchlorid gelöst in 30 ml Dioxan hinzu und rührt anschließend 16h bei Raumtemperatur. Die Reaktionsmischung wird mit 300 ml Wasser verdünnt und zweimal mit 150 ml Methylenchlorid extrahiert. Die organische Phase wird mit Bicarbonatlösung und Wasser gewaschen, über Natriumsulfat getrocknet und eingedampft. Der Rückstand wird über Kieselgel 60 mit Methylenchlorid chromatographiert und ergibt 2,3 g (47% d.Th.) 2,4-Di-[3-Trifluormethylphenyl]-5-diacetylamino-2H-pyridazin-3-on vom Schmelzpunkt 174 °C.

Die in Tabelle 2 als Beispiel 45 aufgeführte Verbindung kann beispielsweise wie folgt hergestellt werden:

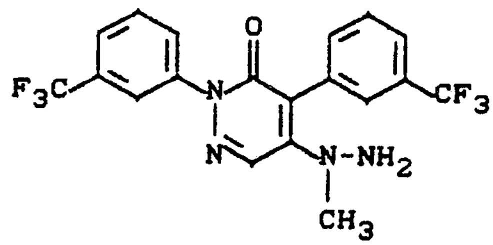

Eine Mischung aus 4,2 g (0,01 Mol) 2,4-Di-[3-Trifluormethylphenyl]-5-chlor-2H-pyridazin-3-on, 1 g (0,02 Mol) Methylhydrazin und 100 ml Ethanol wird 16 Stunden unter Rückfluß erhitzt. Danach wird eingedampft, der Rückstand in Methylenchlorid aufgenommen, mit Wasser gewaschen, die organische Phase über Natriumsulfat getrocknet und eingedampft.

Nach Chromatographie des Rückstands über Kieselgel 60 mit Methylenchlorid erhält man 9,5 g (81% d.Th.) von 2,4-Di-[3-trifluormethylphenyl]-5-[α-methylhydrazinyl]-2H-pyridazin-3-on mit dem Schmelzpunkt 88 °C.

## Herstellung der Ausgangsverbindungen

### Beispiel (IIa)-1

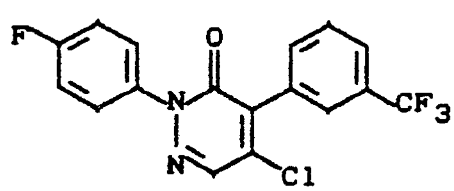

Zu einer Grignard-Lösung, hergestellt aus 1 g (0,04 Mol) Magnesium und 9 g (0,04 Mol) 3-Trifluormethyl-brombenzol in 80 ml Diethylether, tropft man bei Raumtemperatur eine Lösung aus 5,2 g (0,02 Mol) 2-[4-Fluorphenyl]-4,5-dichlor-2H-pyridazin-3-on in 120 ml Diethylether und erhitzt die Reaktionsmischung 16 Stunden am Rückfluß. Anschließend hydrolysiert man mit einer Mischung aus Eis und 1 N - Salzsäure. Die Etherphase wird abgetrennt, mit Wasser gewaschen, über Natriumsulfat getrocknet und eingeengt.

Nach chromatographischer Trennung an Kieselgel 60 mit Methylenchlorid als Laufmittel erhält man 1,8 g (24% d. Theorie) an 2-[4-Fluorphenyl]-4-[3-trifluormethylphenyl]-5-chlor-2H-pyridazin-3-on vom Schmelzpunkt 88-90° C.

In entsprechender Weise und gemäß den allgemeinen Angaben erhält man die folgenden Verbindungen der allgemeinen Formel (IIa)

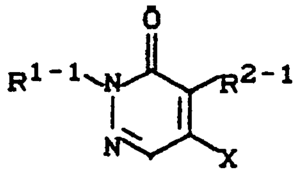

(IIa)

| Bsp.-Nr. | $R^{1-1}$ | $R^{2-1}$ | X | Physikal. Daten |
|---|---|---|---|---|
| (IIa)-2 | $-CH_3$ | 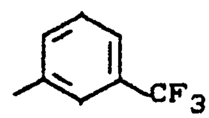 | Cl | 75° C |
| (IIa)-3 | 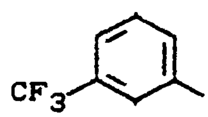 | 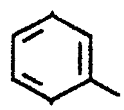 | Cl | 61-63° C |
| (IIa)-4 | 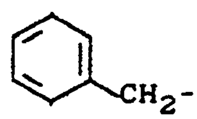 | 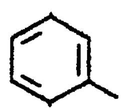 | Cl | 54-56° C |
| (IIa)-5 | $-CH_3$ | 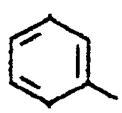 | Cl | $n_D^{20}$ =1,5945 |
| (IIa)-6 | 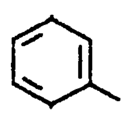 | 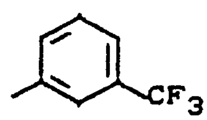 | Cl | 54-57° C |

Anwendungsbeispiel

In dem folgenden Anwendungsbeispiel wurde die nachstehend aufgeführte Verbindung als Vergleichssubstanz eingesetzt,

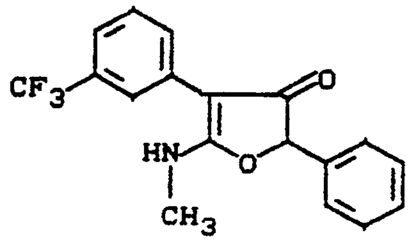

(A)

5-(Methylamino)-2-phenyl-4-[3-(trifluormethyl)-phenyl]-2H-furan-3-on (DE-OS 3422346).

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant Die Wirkstoffkonzentration in der Zubereitung spielt keine Rolle, entscheidend ist nur die Aufwandmenge des Wirkstoffs pro Flächeneinheit Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung

In diesem Test zeigen beispielsweise die Verbindungen gemäß den Herstellungsbeispielen 1, 6, 7, 9, 16, 24, 26, 29, 30, 31, 32, 33, 36, 49, 53, 55 und 56 bei guter Verträglichkeit gegenüber Kulturpflanzen, wie z.B. Weizen, Gerste, Soja und Baumwolle, starke Wirkung gegen Unkräuter.

Beispiel B

Post-emergence-Test

Lösungsmittel: 5 Gewichtsteile Aceton
Emulgator: 1 Gewichtsteil Alkylarylpolyglykolether
Netzmittel: 0,1 % (bezogen auf die gebrauchsfertige Wirkstoffzubereitung) eines Polyoxyethylen-(6)-tridecylethers (mit der Bezeichnung "Renex-36"; CAS 24 938-91-8).

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat unter Zusatz des Netzmittels mit Wasser auf die gewünschte Konzentration.

Mit der Wirkstoffzubereitung spritzt man Testpflanzen, welche eine Höhe von 5-15 cm haben, so, daß die jeweils gewünschten Wirkstoffmengen pro Flächeneinheit ausgebracht werden. Die Konzentration der Spritzbrühe wird so gewählt, daß in 1000 ml Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrollen.
Es bedeuten:
0 % = keine Wirkung (wie unbehandelte Kontrolle)
100 % = totale Vernichtung

In diesem Test zeigen beispielsweise die Verbindungen gemäß den Herstellungsbeispielen 9, 24, 36, 38, 39, 40, 49, 53, 55 und 56 bei guter Verträglichkeit gegenüber Kulturpflanzen, wie z.B. Weizen, Gerste, Soja und Baumwolle, starke Wirkung gegen Unkräuter.

Beispiel C

Entlaubung und Austrocknung der Blätter bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 1 Woche werden der Blattfall und das Austrocknen der Blätter im Vergleich zu den Kontrollpflanzen bonitiert.

In diesem Test zeigt beispielsweise die Verbindung gemäß Herstellungsbeispiel 1 starke pflanzenwuchsregulierende Wirkung.

**Ansprüche**

1. 2H-Pyridazinon-Derivate der allgemeinen Formel (I),

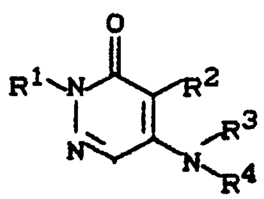

(I)

in welcher
$R^1$ für Alkyl, Alkoxyalkyl, Alkylthioalkyl, Aminoalkyl, Alkylaminoalkyl, Dialkylaminoalkyl, Hydroxyalkyl, Halogenalkyl, Cyanalkyl, Alkenyl, Halogenalkenyl, Alkoxycarbonylalkyl oder für jeweils gegebenenfalls substituiertes Cycloalkyl, Cycloalkylalkyl, Phenyl oder Phenylalkyl steht,
$R^2$ für Alkyl oder für jeweils gegebenenfalls substituiertes Phenyl, Phenoxy oder Phenylalkyl steht,
$R^3$ für Wasserstoff oder für jeweils gegebenenfalls substituiertes Alkyl, Cycloalkyl oder Alkylcarbonyl steht und
$R^4$ für Wasserstoff, Hydroxy, Amino, Aminocarbonyl (Carbamoyl), für Alkoxy, Alkylamino oder Di alkylamino, für jeweils gegebenenfalls substituiertes Alkyl, Cycloalkyl, Alkylaminocarbonyl, Dialkylaminocarbonyl, Alkylcarbonyloxy, Alkylcarbonylamino, Alkylcarbonylalkylamino, Bis-(alkylcarbonyl)-amino, Alkoxycarbonyl, Alkoxycarbonylcarbonyl, Formyl oder Alkylcarbonyl steht.

2. 2H-Pyridazinon-Derivate der allgemeinen Formel (I) gemäß Anspruch 1, dadurch gekennzeichnet, daß darin
$R^1$ für geradkettiges oder verzweigtes Alkyl mit 1 bis 6 Kohlenstoffatomen, geradkettiges oder verzweigtes Alkoxyalkyl oder Alkylthioalkyl mit jeweils 1 bis 4 Kohlenstoffatomen im Alkyl- und Alkoxy- bzw. Alkylthioteil, geradkettiges oder verzweigtes Aminoalkyl, Alkylaminoalkyl, Dialkylaminoalkyl, Hydroxyalkyl, Halogenalkyl oder Cyanalkyl mit jeweils 1 bis 4 Kohlenstoffatomen in den jeweiligen Alkylteilen und 1-4 Halogenatomen im Halogenalkylteil, geradkettiges oder verzweigtes Alkenyl oder Halogenalkenyl mit jeweils 2-6 Kohlenstoffatomen im Alkenylteil und 1-4 Halogenatomen im Halogenalkenylteil geradkettiges oder verzweigtes Alkoxycarbonylalkyl mit jeweils 1-4 Kohlenstoffatomen im Alkoxy- und Alkylteil, gegebenenfalls einfach bis vierfach gleich oder verschieden im Cycloalkylteil substituiertes Cycloalkyl oder Cycloalkylalkyl mit jeweils 3-6 Kohlenstoffatomen im Cycloalkylteil und gegebenenfalls 1-3 Kohlenstoffatomen im geradkettigen oder verzweigten Alkylteil steht (wobei als Substituenten jeweils infrage kommen: Halogen, jeweils geradkettiges oder verzweigtes Alkyl, Alkoxy, Halogenalkyl oder Halogenalkoxy mit jeweils 1 bis 4 Kohlenstoffatomen und gegebenenfalls 1 bis 9 gleichen oder verschiedenen Halogenatomen); oder für gegebenenfalls einfach bis dreifach gleich oder verschieden im Phenylteil substituiertes Phenyl oder Phenylalkyl mit gegebenenfalls 1-3 Kohlenstoffatomen im geradkettigen oder verzweigten Alkylteil steht (wobei als Substituenten jeweils infrage kommen: Halogen, Cyano, Nitro, jeweils geradkettiges oder verzweigtes Alkyl oder Alkoxy mit jeweils 1-6 Kohlenstoffatomen, Halogenalkyl oder Halogenalkoxy mit jeweils 1 oder 2 Kohlenstoffatomen

und 1-5 gleichen oder verschiedenen Halogenatomen):

$R^2$ für geradkettiges oder verzweigtes Alkyl mit 1-6 Kohlenstoffatomen oder für gegebenenfalls einfach bis dreifach gleich oder verschieden im Phenylteil substituiertes Phenyl, Phenoxy oder Phenylalkyl mit gegebenenfalls 1-3 Kohlenstoffatomen im geradkettigen oder verzweigten Alkylteil steht) wobei als Substi tuenten jeweils infrage kommen: Halogen, Cyano, Nitro, jeweils geradkettiges oder verzweigtes Alkyl oder Alkoxy mit jeweils 1-6 Kohlenstoffatomen, jeweils geradkettiges oder verzweigtes Halogenalkyl oder Halogenalkoxy mit jeweils 1-2 Kohlenstoffatomen und 1-5 gleichen oder verschiedenen Halogenatomen);

$R^3$ für Wasserstoff, für jeweils geradkettiges oder verzweigtes Alkyl mit 1-4 Kohlenstoffatomen (welches gegebenenfalls durch 1-9 gleiche oder verschiedene Halogenatome und/oder durch Alkoxy mit 1-4 Kohlenstoffatomen substituiert ist), oder für gegebenenfalls einfach bis vierfach gleich oder verschieden substituiertes Cycloalkyl mit 3-6 Kohlenstoffatomen steht (wobei als Substituenten jeweils infrage kommen: Halogen, sowie jeweils geradkettiges oder verzweigtes Alkyl, Alkoxy, Halogenalkyl oder Halogenalkoxy mit jeweils 1-4 Kohlenstoffatomen und gegebenenfalls 1-9 gleichen oder verschiedenen Halogenatomen), oder für geradkettiges oder verzweigtes und gegebenenfalls substituiertes Alkylcarbonyl mit 1-4 Kohlenstoffatomen im Alkylteil steht (wobei als Substituenten 1-3 gleiche oder verschiedene Halogenatome infrage kommen),

und

$R^4$ für Wasserstoff, Hydroxy, Amino, Aminocarbonyl für jeweils geradkettiges oder verzweigtes Alkoxy, Alkylamino oder Dialkylamino mit 1-4 Kohlenstoffatomen in den jeweiligen Alkylteilen, für jeweils geradkettiges oder verzweigtes Alkyl mit 1-4 Kohlenstoffatomen (welches gegebenenfalls durch 1-9 gleiche oder verschiedene Halogenatome und/oder Hydroxygruppen bzw. durch Alkoxy mit 1-4 Kohlenstoffatomen substituiert ist) oder für gegebenenfalls einfach bis vierfach gleich oder verschieden substituiertes Cycloalkyl mit 3-6 Kohlenstoffatomen (wobei als Substituenten jeweils infrage kommen: Halogen, sowie jeweils geradkettiges oder verzweigtes Alkyl, Alkoxy, Halogenalkyl oder Halogenalkoxy mit jeweils 1-4 Kohlenstoffatomen und gegebenenfalls 1-9 gleichen oder verschiedenen Halogenatomen), für jeweils geradkettiges oder verzweigtes Alkylaminocarbonyl oder Dialkylaminocarbonyl mit 1-4 Kohlenstoffatomen in den jeweiligen Alkylteilen, für im Alkylcarbonylteil gegebenenfalls jeweils 1-3fach durch Halogen substituiertes Alkylcarbonyloxy, Alkylcarbonylamino, Alkylcarbonyl-alkyl-amino oder Bis-(alkylcarbonyl)-amino mit 1-4 Kohlenstoffatomen in den jeweiligen Alkylteilen, für jeweils geradkettiges oder verzweigtes und gegebenenfalls substituiertes Alkoxycarbonyl oder Alkoxycarbonylcarbonyl mit 1-4 Kohlen stoffatomen im Alkylteil oder für geradkettiges oder verzweigtes und gegebenenfalls substituiertes Alkylcarbonyl mit 1-4 Kohlenstoffatomen im Alkylteil steht (wobei als Substituenten 1-3 gleiche oder verschiedene Halogenatome infrage kommen).

3. 2H-Pyridazinon-Derivate der allgemeinen Formel (I) gemäß Anspruch 1, dadurch gekennzeichnet, daß darin

$R^1$ für Methyl, Ethyl, n- oder i-Propyl, n-, i-, s- oder t-Butyl, Methoxymethyl, Ethoxymethyl, Methoxyethyl oder Ethoxyethyl, Methylthiomethyl, Ethylthiomethyl, Methylthioethyl, Ethylthioethyl, Aminomethyl, Aminoethyl, Methylaminomethyl, Methylaminoethyl, Dimethylaminomethyl, Dimethylaminoethyl Ethylaminomethyl, Ethylaminoethyl, Diethylaminomethyl, Diethylaminoethyl, Hydroxymethyl, Hydroxyethyl, 1-3 Fluor-und/oder Chloratome enthaltendes Halogenmethyl, Halogenethyl, n- oder i-Halogenpropyl, n- oder i-Halogenbutyl, Cyanmethyl, Cyanethyl, Allyl, n- oder i-Butenyl, n-oder i-Pentenyl, 2-Fluorpropen-3-yl, 2-Chlorpropen-3-yl, 1-Chlorpropen-3-yl 1,1-Dichlorpropen-3-yl, 1-Fluorpropen-3-yl, 1,1-Difluorpropen-3-yl, 1,2-Dichlorpropen-3-yl, 1,2-Difluorpropen-3-yl, 1,1,2-Trichlorpropen-3-yl, für Methoxycarbonylmethyl,
Methoxycarbonylethyl, Ethoxycarbonylmethyl oder Ethoxycarbonylethyl, oder für gegebenenfalls ein-bis vierfach gleich oder verschieden im Cycloalkylteil substituiertes Cyclopentyl, Cyclohexyl, Cyclopropylmethyl, Cyclopentylmethyl, Cyclopentylethyl, Cyclohexylmethyl oder Cyclohexylethyl steht (wobei als Substituenten jeweils infrage kommen:

Fluor, Chlor, Brom, Methyl, Ethyl, Methoxy, Ethoxy, Halogenmethyl, Halogenethyl, Halogenmethoxy oder Halogenethoxy, wobei Halogen bevorzugt für Fluor und/oder Chlor steht); weiterhin für gegebenenfalls ein- oder zweifach gleich oder verschieden im Phenylteil substituiertes Phenyl, Benzyl oder Phenethyl steht (wobei als Substituenten jeweils infrage kommen: Fluor, Chlor, Brom, Cyano, Nitro, Methyl, Ethyl, n-oder i-Propyl, n-, i-, s-oder t-Butyl, Methoxy, Ethoxy, Trifluormethyl, Difluormethyl sowie Trifluormethoxy);

$R^2$ für Methyl, Ethyl, n- oder i-Propyl, n-, i-, s-oder t-Butyl, oder für gegebenenfalls ein-bis zweifach gleich oder verschieden im Phenylteil substituiertes Phenyl, Phenoxy, Benzyl oder Phenethyl steht (wobei als Substituenten jeweils infrage kommen: Fluor, Chlor, Brom, Cyano, Nitro, Methyl, Ethyl, n- oder i- Propyl, n-, i-, s- oder t-Butyl, Methoxy, Ethoxy, Trifluormethyl, Difluormethyl sowie Trifluormethoxy);

$R^3$ für Wasserstoff, Methyl, Ethyl, n- oder i-Propyl, Halogenmethyl oder Halogenethyl (wobei Halogen bevorzugt für Fluor und/oder Chlor steht), Methoxymethyl, Methoxyethyl, Ethoxymethyl, Ethoxyethyl, für gegebenenfalls ein-oder zweifach gleich oder verschieden substituiertes Cyclopropyl (wobei als Substituenten jeweils infrage kommen: Fluor, Chlor, Brom, Methyl, Ethyl) oder für Acetyl, Formyl, Propionyl, Trifluoracetyl, Trichloracetyl oder Chloracetyl steht, und
$R^4$ für Wasserstoff, Hydroxy, Amino, Methoxy, Ethoxy, Methylamino, Ethylamino, Dimethylamino, Diethylamino, Methyl, Ethyl, n- oder i-Propyl, Hydroxymethyl, Hydroxyethyl, Methylaminocarbonyl, Ethylaminocarbonyl, Dimethylaminocarbonyl, Methylethylaminocarbonyl oder Diethylaminocarbonyl, Acetyloxy, Trifluoracetyloxy, Acetylamino, Trifluoracetylamino, Acetylmethylamino, Trifluoracetyl-methylamino, Di-acetylamino, Di-trifluoracetylamino, Methoxycarbonyl, Ethoxycarbonyl, Methoxycarbonylcarbonyl, Ethoxycarbonylcarbonyl, Formyl, Acetyl, Propionyl, Di-fluoracetyl, Trifluoracetyl, Chloracetyl, Dichloracetyl oder Trichloracetyl steht.

4. 2H-Pyridazinon-Derivate der allgemeinen Formel (I) gemäß Anspruch 1, dadurch gekennzeichnet, daß darin
$R^1$ für Methyl, Ethyl, n- oder i-Propyl, n-, i-, s-oder t-Butyl, Methoxymethyl, Ethoxymethyl, Methoxyethyl, Methylthiomethyl, Ethylthiomethyl, Methylthioethyl, Dimethylaminomethyl, Hydroxymethyl, 1,1,1-Trifluorethyl, Allyl, n- oder i-Butenyl, n-oder i-Pentenyl, 2-Fluorpropen-3-yl, 2-Chlorpropen-3-yl, 1-Chlorpropen-3-yl, 1,1-Dichlorpropen-3-yl, 1-Fluorpropen-3-yl, 1,1-Difluorpropen-3-yl, 1,2-Dichlorpropen-3-yl, 1,2-Difluorpropen-3-yl, 1,1,2-Trichlorpropen-3-yl, Cyclopropylmethyl, 1,1-Dichlorcyclopropylmethyl, 1,1-Difluorcyclopropylmethyl, 1,1-Dimethylcyclopropylmethyl, 1,1-Dimethyl-2,2-dichlorcyclopropylmethyl, für jeweils gegebenenfalls einfach oder zweifach substituiertes Cyclopentyl oder Cyclohexyl steht (wobei als Substituenten Methyl und/oder Ethyl infrage kommen), oder für gegebenenfalls einfach oder zweifach gleich oder verschieden substituiertes Phenyl steht (wobei als Substituenten infrage kommen: Fluor, Chlor, Brom, Methyl, Ethyl, Methoxy, Ethoxy, Trifluormethyl oder Trifluormethoxy);
$R^2$ für Methyl, Ethyl, n- oder i-Propyl, n-, i-, s-oder t-Butyl oder für einfach oder zweifach substituiertes Phenyl steht, wobei als Substituenten infrage kommen: Fluor, Chlor, Brom, Methyl, Ethyl, Methoxy, Ethoxy, Trifluormethyl oder Trifluormethoxy,
$R^3$ für Wasserstoff, Methyl, Ethyl, n- oder i-Propyl, Acetyl oder Trifluoracetyl steht und
$R^4$ für Hydroxy, Amino, Methylamino, Dimethylamino, Methyl, Ethyl, n- oder i-Propyl, Acetyloxy, Trifluoracetyloxy, Acetylamino, Trifluoracetylamino, Acetylmethylamino, Trifluoracetylmethylamino, Diacetylamino, Di-trifluoracetylamino, Acetyl oder Trifluoracetyl steht.

5. Verfahren zur Herstellung von 2H-Pyridazinon-Derivaten der allgemeinen Formel (I) gemäß Anspruch 1, dadurch gekennzeichnet, daß man
(a) 2H-5-Halogen-pyridazinon-Derivate der allgemeinen Formel (II),

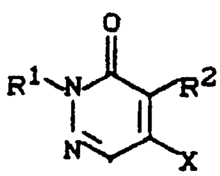

(II)

in welcher
$R^1$ und $R^2$ die in Anspruch 1 angegebene Bedeutung haben und
X für Halogen steht,
mit Aminen der allgemeinen Formel (III),

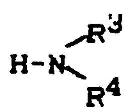

(III)

in welcher
$R^3$ und $R^4$ die in Anspruch 1 angegebene Bedeutung haben,
gegebenenfalls in Gegenwart eines Verdünnungsmittels und gegebenenfalls in Gegenwart eines basischen Reaktionshilfsmittels (Säurebindemittels) umsetzt, oder daß man
(b) die nach Verfahren (a) erhaltenen erfindungsgemäßen Verbindungen der allgemeinen Formel (Ia),

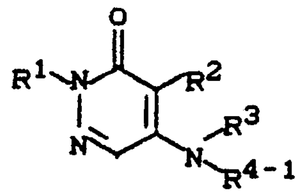

(Ia)

in welcher
$R^1$, $R^2$ und $R^3$ die oben angegebene Bedeutung haben und
$R^{4-1}$ für Wasserstoff, Hydroxy, Amino oder Alkylamino steht,
mit Acylierungsmitteln der allgemeinen Formel (IV),
$R^{4-2}$-Z (IV)
in welcher
$R^{4-2}$ für jeweils gegebenenfalls substituiertes (Di)Alkylaminocarbonyl, Alkoxycarbonyl, Alkoxycarbonylcarbonyl oder Alkylcarbonyl steht und
Z für eine elektronenanziehende Abgangsgruppe steht,
gegebenenfalls in Gegenwart eines Verdünnungsmittels und gegebenenfalls in Gegenwart eines basischen Reaktionshilfsmittels (Säurebindemittels) umsetzt.

6. Herbizide und pflanzenwachstumsregulierende Mittel, gekennzeichnet durch einen Gehalt an mindestens einem 2H-Pyridazinon-Derivat der allgemeinen Formel (I) gemäß Anspruch 1.

7. Verfahren zur Bekämpfung von Unkraut, dadurch gekennzeichnet, daß man ein 2H-Pyridazinon-Derivat der Formel (I) gemäß Anspruch 1 auf die Unkräuter oder ihren Lebensraum einwirken läßt.

8. Verwendung von 2H-Pyridazinon-Derivaten der Formel (I) gemäß Anspruch 1 zur Bekampfung von unerwünschtem Pflanzenwachstum und/oder zur Regulierung des Pflanzenwachstums.

9. Verfahren zur Herstellung von herbiziden bzw. pflanzenwachstumsregulierenden Mitteln, dadurch gekennzeichnet, daß man 2H-Pyridazinon-Derivate der Formel (I) gemäß Anspruch 1 mit Streckmitteln und/oder oberflächenaktiven Stoffen vermischt.

10. 2H-5-Halogen-pyridazinon-Derivate der allgemeinen Formel (IIa)

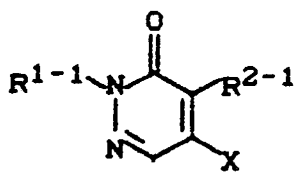

(IIa)

in welcher
$R^{1-1}$ für Alkyl, Alkoxyalkyl, Alkylthioalkyl, Aminoalkyl, (Di)alkylaminoalkyl, Hydroxyalkyl, Halogenalkyl, Alkenyl, Halogenalkenyl, für jeweils gegebenenfalls substituiertes Cycloalkyl, Cycloalkylalkyl, Phenyl oder Phenylalkyl steht;
$R^{2-1}$ für jeweils gegebenenfalls substituiertes Phenyl oder Phenylalkyl steht und
X für Halogen steht.
$R^3$ für Wasserstoff oder für jeweils gegebenenfalls substituiertes Alkyl, Cycloalkyl oder Alkylcarbonyl steht und
$R^4$ für Wasserstoff, Hydroxy, Amino, Aminocarbonyl (Carbamoyl), für Alkoxy, Alkylamino oder Dialkylamino, für jeweils gegebenenfalls substituiertes Alkyl, Cycloalkyl, Alkylaminocarbonyl, Dialkylaminocarbonyl, Alkylcarbonyloxy, Alkylcarbonylamino, Alkylcarbonylalkylamino, Bis-(alkylcarbonyl)-amino, Alkoxycarbonyl, Alkoxycarbonylcarbonyl, Formyl oder Akylcarbonyl steht,
Verfahren zu ihrer Herstellung und ihre Verwendung als Herbizide.